# EUROPÄISCHE PATENTSCHRIFT

(11) **EP 1 647 326 B1**
(45) Veröffentlichungstag und Bekanntmachung des Hinweises auf die Patenterteilung: **14.09.2011**
(21) Anmeldenummer: 05027659.1
(22) Anmeldetag: 19.03.1999
(51) Int. Cl.: A61K 9/50, B01J 13/02

(54) **Verfahren zur Herstellung von Kapseln mit einer Polyelektrolythülle**
Process for preparing capsules having a polyelectrolyte shell
Procédé de préparation de capsules ayant une enveloppe de polyélectrolyte

(30) Priorität: 19.03.1998 DE 19812083; 15.07.1998 EP 98113181; 22.02.1999 DE 19907552
(43) Veröffentlichungstag der Anmeldung: 19.04.2006
(62) Teilanmeldung aus: 99911804.5
(73) Patentinhaber: Max-Planck-Gesellschaft zur Förderung der Wissenschaften e.V., 80539 München (DE)
(72) Erfinder: Donath, Edwin, Prof. Dr., 04316 Leipzig (DE); Sukhorukov, Gleb, Dr., Moscow 115280 (RU); Lerche, Karl-Heinz, Dr., 10243 Berlin (DE); Voigt, Andreas, Dr., 12621 Berlin (DE); Bäumler, Hans, Dr., 12437 Berlin (DE); Caruso, Frank, Prof.Dr., Preston Victoria 3072 (AU); Möhwald, Helmuth, Prof. Dr., 55411 Bingen (DE)
(74) Vertreter: Kilger, Christian

(56) Entgegenhaltungen:
- EP-A2- 1 867 325
- DE-A1- 4 312 970
- GB-A- 2 145 992
- US-A- 4 663 286
- US-A- 5 487 390
- CARUSO F ET AL: "INFLUENCE OF POLYELECTROLYTE MULTILAYER COATINGS ON FOERSTER RESONANCE ENERGY TRANSFER BETWEEN 6-CARBOXYFLUORESCEIN AND RHODAMINE B-LABELED PARTICLES IN AQUEOUS SOLUTION" JOURNAL OF PHYSICAL CHEMISTRY. B, MATERIALS, SURFACES, INTERFACES AND BIOPHYSICAL, WASHINGTON, DC, US, Bd. 102, 24. Februar 1998 (1998-02-24), Seiten 2011-2016, XP000852731 ISSN: 1089-5647
- SUKHORUKOV G B; ET AL: "LAYER-BY-LAYER SELF ASSEMBLY OF POLYELECTROLYTES ON COLLOIDAL PARTICLES" COLLOIDS AND SURFACES. A, PHYSICACHEMICAL AND ENGINEERING ASPECTS,, Bd. 137, 1998, Seiten 253-266, XP000852906

## Beschreibung

Die Erfindung betrifft Nano- bzw. Mikrokapseln, die eine Polyelektrolythülle umfassen, ein Verfahren zur Herstellung dieser Kapseln sowie ihre Verwendung.

Mikrokapseln sind in verschiedenen Ausführungsformen bekannt und werden insbesondere für die kontrollierte Freisetzung und den zielgerichteten Transport von pharmazeutischen Wirkstoffen sowie zum Schutz von empfindlichen Wirkstoffen, wie etwa Enzymen und Proteinen verwendet (siehe z.B. D.D.Lewis, "Biodegradable Polymers and Drug Delivery Systems", M.Chasin and R.Langer, Hrsg. (Marcel Decker, New York, 1990); J.P.McGee et al., J.Control.Release 34 (1995), 77).

Mikrokapseln können durch mechanisch-physikalische Verfahren, wie z.B. Versprühen und nachfolgende Beschichtung hergestellt werden. Die auf diese Weise erhältlichen Mikrokapseln weisen jedoch eine Reihe von Nachteilen auf. Insbesondere ist es mit den bekannten mechanisch-physikalischen Verfahren nicht möglich, Mikrokapseln mit einer Größe von < 10 µm (Durchmesser) herzustellen. Vielmehr sind nur Mikrokapseln mit relativ großen Durchmessern erhältlich, deren Anwendungsbereich jedoch aufgrund ihrer Größe beschränkt ist. Weiterhin wird bei den bekannten mechanisch-physikalischen Verfahren keine monodisperse Kapselverteilung, sondern vielmehr eine uneinheitliche Verteilung von Kapseln verschiedener Größe erhalten. Auch dies ist für viele Anwendungen, bei denen die Größe der Kapsel wichtig ist, nachteilig.

Neben den mechanisch-physikalischen Verfahren sind auch chemische Verfahren zur Herstellung von Mikrokapseln bekannt. So können Mikrokapseln durch Grenzflächenpolymerisation bzw. -kondensation oder durch Polymerphasentrennung aus einem Polymer/Lösungsmittelgemisch hergestellt werden (B.Miksa et al., Colloid Polym.Sci.273 (1995), 47; G.Crotts et al., J.Control.Release 35 (1995), 91; S.L.Regen et al., J.Am.Chem.Soc.106 (1984), 5756). Aber auch die durch bekannte chemische Verfahren hergestellten Mikrokapseln weisen eine Reihe von Nachteilen auf. Insbesondere sind eine hohe Polydispersität, eine ungleichmäßige Umhüllung sowie oftmals eine Verfestigung des Kerns zu beobachten. Ein weiterer wesentlicher Nachteil der bekannten chemischen Verfahren liegt in der Verwendung von organischen Lösungsmitteln und polymerisierbaren organischen Monomeren, was zu beträchtlichen Beschränkungen hinsichtlich der verwendbaren einzukapselnden Wirkstoffe führt. Insbesondere die oftmals dadurch notwendige Verwendung von mit Wasser nicht mischbaren organischen Flüssigkeiten als Kernmaterial schränkt den Anwendungsbereich solcher Mikrokapseln, gerade im Hinblick auf Proteine oder Enzyme drastisch ein.

Ein weiteres System, das zur Einkapselung von anorganischen und organischen Materialien verwendet wurde, sind Lipidliposomen (D.D.Lasic, "Liposomes: From Physics to Applications" (Elsevier, Amsterdam, 1993); S.L.Regen et al., J.Am.Chem.Soc.106 (1984), 2446). Die Einkapselung von Wirkstoffen in Lipidliposomen gestattet die Herstellung von Mikrokapseln unter relativ schonenden Bedingungen, weshalb Liposomen als Trägersysteme für verschiedene pharmazeutische und kosmetische Wirkstoffe verwendet werden. Die biologische, chemische und mechanische Stabilität solcher Liposomkapseln ist jedoch sehr gering, was die allgemeine Verwendbarkeit solcher Kapseln limitiert. Einen weiteren Nachteil stellt die geringe Permeabilität von Liposomenkapseln, insbesondere für polare Moleküle, dar, die einen Stoffaustausch mit dem Umgebungsmedium verhindert.
Bei einem weiteren Verfahren zur Herstellung von Mikrokapseln werden zunächst Mischungen aus dem einzuschließenden Material und einem mit z.B. Ca²⁺-Ionen verfestigbaren Polyelektrolytbestandteil gebildet. Diese Mischung wird in Form kleinster Tröpfchen in ein Ca²⁺-Bad eingebracht, wobei sich eine Gelstruktur bildet, die dann in weiteren Verfahrensschritten mit einer Polyelektrolytkapsel umgeben werden kann. Eine Weiterentwicklung solcher Verfahren wird in DE 33 06 259 A1 beschrieben, wobei auf die Verwendung von Ca²⁺ verzichtet werden kann. Der hauptsächliche Nachteil dieser Verfahren besteht darin, daß die Größe der herstellbaren Mikrokapseln nach unten auf etwa 50 µm (Durchmesser) begrenzt ist und die Wandstärke der erhaltenen Mikrokapseln mindestens 100 nm beträgt.

In DE-A-40 26 978 ist ein Verfahren zur Beschichtung von flächigen Trägern beschrieben, wobei ein Träger so modifiziert wird, daß er flächenweit Ionen oder ionisierbare Verbindungen mit gleichsinniger Ladung trägt und eine oder mehrere Schichten aus organischen Materialien, die in jeder Schicht gleichsinnig geladene Ionen enthalten, aus einer Lösung solcher organischer Materialien auf den modifizierten Träger aufgetragen wird, wobei das organische Material für die erste Schicht Ionen mit entgegengesetztem Ladungsinn zum Ladungssinn der lonenmodifizierung des Trägers aufweist und im Falle von mehreren Schichten abwechselnd weitere Schichten mit Ionen mit dem jeweils entgegengesetzten Ladungssinn zur vorhergehenden in gleicher Weise wie die erste Schicht aufgetragen werden. Als Träger werden anorganische oder organische Trägermaterialien mit ebenmäßiger Oberfläche offenbart. Es findet sich keinerlei Hinweis auf die Verwendung von Mikropartikeln als Trägermaterialien oder auf eine Auflösung der Trägermaterialien nach der Beschichtung.

Eine Aufgabe der Erfindung bestand deshalb darin, Kapseln mit einem geringen Durchmesser bereitzustellen, in denen Materialen, wie etwa Makromoleküle, Präzipitate, Flüssigkeiten oder Gase, eingeschlossen werden können. Die Kapseln sollten weiterhin eine hohe Stabilität und Hüllen mit geringer Wandstärke aufweisen, die insbesondere für Ionen und kleine Moleküle durchlässig sind.

Die Aufgabe wird erfindungsgemäß gelöst durch ein Verfahren zur Herstellung von Kapseln mit einer Polyelektrolythülle, die mehrere Polyelektrolytschichten umfasst, und einen Durchmesser bis zu 10 µm aufweist, umfassend die Schritte:
a) Bereitstellen einer wässrigen Dispersion von Templatpartikeln geeigneter Größe,
b) Herstellen einer Hülle um die Partikel durch Aufbringen von Polyelektrolyten auf die Templatpartikel und
c) zumindest teilweise Desintegration der Templatpartikel.

Ein weiterer Gegenstand der Erfindung sind Kapseln, die durch das erfindungsgemäße Verfahren erhältlich sind und eine von den Templatpartikeln vorgegebene äußere Form aufweisen.

Überraschenderweise wurde festgestellt, daß bei Beschichtung von Templatpartikeln mit einer Polyelektrolythülle und anschließender Desintegration der Templatpartikel Kapseln mit definierten inneren und äußeren Hülleigenschaften und mit selektiv steuerbaren Permeabilitätseigenschaften erhalten werden können. Unter einer Polyelektrolythülle wird eine Hülle mit einem Gehalt an Polyelektrolyten verstanden. Bevorzugt besteht die Polyelektrolythülle zu mindestens 50% insbesondere mindestens 60% und besonders bevorzugt zu mindestens 80% aus Polyelektrolyten. Die erfindungsgemäßen Kapseln gestatten den Einschluß auch empfindlicher Moleküle unter schonenden Bedingungen, z.B. in wäßrigen Lösungen. Die Kapselwand ist eine Polyelektrolythülle, die den Stoffaustausch im Hinblick auf niedermolekulare Stoffe und Ionen mit der Umgebung ermöglicht, aber gleichzeitig makromolekulare Stoffe zurückhält. Die chemischen und physikalischen Eigenschaften der als Kapselwand dienenden Polyelektrolythülle können in weiten Grenzen durch den Aufbau und die Zusammensetzung der Hülle sowie durch Umgebungsparameter gesteuert werden. So können die erfindungsgemäßen Kapseln beispielsweise als Transporträume dienen, wobei hier die Parameter der äußeren Schicht den Transport an vorgegebene Zielorte, z.B. im Organismus, bestimmen.

Die erfindungsgemäßen Kapseln umfassen Mikrokapseln mit einem Durchmesser von ≤ 10 µm, besonders bevorzugt ≤5µm und am meisten bevorzugt ≤ 2 µm sowie Nanokapseln mit einem Durchmesser von ≥ 10 nm bis < 1000 nm.

Die Hülle der Kapseln weist mehrere Polyelektrolytschichten auf. Unter Polyelektrolyten werden allgemein Polymere mit ionisch dissoziierbaren Gruppen, die Bestandteil oder Substituent der Polymerkette sein können, verstanden. Üblicherweise ist die Zahl dieser ionisch dissoziierbaren Gruppen in Polyelektrolyten so groß, daß die Polymeren in der dissoziierten Form (auch Polyionen genannt) wasserlöslich sind. Hierin werden unter dem Begriff Polyelektrolyte auch Ionomere verstanden, bei denen die Konzentration der ionischen Gruppen für eine Wasserlöslichkeit nicht ausreichend sind, die jedoch genügend Ladungen aufweisen, um eine Selbstassemblierung einzugehen. Bevorzugt umfasst die Hülle "echte" Polyelektrolyte. Je nach Art der dissoziierbaren Gruppen werden Polyelektrolyte in Polysäuren und Polybasen unterteilt. Aus Polysäuren entstehen bei der Dissoziation unter Abspaltung von Protonen Polyanionen, die sowohl anorganische als auch organische Polymere sein können. Beispiele für Polysäuren sind Polyphosphorsäure, Polyvinylschwefelsäure, Polyvinyl-sulfonsäure, Polyvinylphosphonsäure und Polyacrylsäure. Beispiele für die entsprechenden Salze, die auch als Polysalze bezeichnet werden, sind Polyphosphat, Polysulfat, Polysulfonat, Polyphosphonat und Polyacrylat.

Polybasen enthalten Gruppen, die in der Lage sind, Protonen, z.B. durch Reaktion mit Säuren unter Salzbildung, aufzunehmen. Beispiele für Polybasen mit ketten - bzw. seitenständigen dissoziierbaren Gruppen sind Polyethylenimin, Polyvinylamin und Polyvinylpyridin. Polybasen bilden durch Aufnahme von Protonen Polykationen.

Erfindungsgemäß geeignete Polyelektrolyte sind sowohl Biopolymere, wie etwa Alginsäure, Gummi arabicum, Nucleinsäuren, Pektine, Proteine und andere, sowie chemisch modifizierte Biopolymere, wie etwa ionische oder ionisierbare Polysaccharide, z.B. Carboxymethylcellulose, Chitosan und Chitosansulfat, Ligninsulfonate sowie synthetische Polymere, wie etwa Polymethacrylsäure, Polyvinylsulfonsäure, Polyvinylphosphonsäure und Polyethylenimin.

Es können lineare oder verzweigte Polyelektrolyte eingesetzt werden. Die Verwendung verzweigter Polyelektrolyte führt zu weniger kompakten Polyelektrolytmultifilmen mit einem höheren Grad der Wandporosität. Zur Erhöhung der Kapselstabilität können Polyelektrolytmoleküle innerhalb oder/und zwischen den einzelnen Schichten vernetzt werden, z.B. durch Crosslinking von Aminogruppen mit Aldehyden. Weiterhin können amphiphile Polyelektrolyte, z.B. amphiphile Block- oder Randomcopolymere mit partiellem Polyelektrolytcharakter zur Verringering der Permeabilität gegenüber polaren kleinen Molekülen eingesetzt werden. Solche amphiphilen Copolymere bestehen aus Einheiten unterschiedlicher Funktionalität, z.B. einerseits sauren oder basischen Einheiten und andererseits hydrophoben Einheiten wie Styrolen, Dienen oder Siloxanen etc. die als Blöcke oder statistisch verteilt über das Polymer angeordnet sein können. Durch Verwendung von Copolymeren, die als Funktion äußerer Bedingungen ihre Struktur ändern, können die Kapselwände bezüglich ihrer Permeabilität oder anderer Eigenschaften definiert gesteuert werden. Hierzu bieten sich beispielsweise Copolymere mit einem Poly(N-isopropyl-acrylamid)-Anteil, z.B. Poly(N-isopropylacrylamid-acrylsäure) an, die über das Gleichgewicht von Wasserstoffbrückenbindungen ihre Wasserlöslichkeit als Funktion der Temperatur ändern, was mit einer Quellung einhergeht. Durch Verwendung von unter bestimmten Bedingungen abbaubaren, z.B. photo-, säure-, base- oder salzlabilen Polyelektrolyten kann über die Auflösung der Kapselwände die Freisetzung von eingeschlossenen Wirkstoffen gesteuert werden. Weiterhin können für bestimmte Anwendungsmöglichkeiten auch leitende Polyelektrolyten oder Polyelektrolyten mit optisch aktiven Gruppen als Kapselkomponenten verwendet werden.

Durch geeignete Wahl der Polyelektrolyte ist es möglich, die Eigenschaften und Zusammensetzung der Polyelektrolythülle der erfindungsgemäßen Kapseln definiert einzustellen. Insbesondere bei schichtweise aufgebauten Polyelektrolythüllen kann die Zusammensetzung der Hüllen durch die Wahl der Substanzen beim Schichtaufbau in weiten Grenzen variiert werden. Grundsätzlich ergeben sich keine Einschränkungen hinsichtlich der zu verwendenden Polyelektrolyte bzw. Ionomere, solange die verwendeten Moleküle eine genügend hohe Ladung aufweisen oder/und die Fähigkeit besitzen, über andere Wechselwirkungsarten, wie beispielsweise Wasserstoffbrückenbindungen und/oder hydrophobe Wechselwirkungen, eine Bindung mit der darunter liegenden Schicht einzugehen.

Geeignete Polyelektrolyte sind somit sowohl niedermolekulare Polyelektrolyte bzw. Polyionen als auch makromolekulare Polyelektrolyte, beispielsweise Polyelektrolyte biologischer Herkunft.

Von besonderer Bedeutung für die Verwendung der Kapseln ist die Permeabilität der Hüllwand. Wie bereits oben ausgeführt, ermöglicht die Vielzahl der zur Verfügung stehenden Polyelektrolyte die Herstellung einer Vielzahl von Hüllkompositionen mit unterschiedlichen Eigenschaften. Insbesondere kann die elektrische Ladung der Außenhülle dem Anwendungszweck angepaßt werden. Zudem kann die Innenhülle an jeweils verkapselte Wirkstoffe angepaßt werden, wodurch z.B. eine Stabilisierung des Wirkstoffs erzielt werden kann. Daneben kann auch die Permeabilität der Hüllwand durch die Wahl der Polyelektrolyte in der Hülle und durch die Wanddicke sowie die Umgebungsbedingungen beeinflußt werden. Dadurch ist eine selektive Gestaltung der Permeabilistätseigenschaften sowie eine definierte Veränderung dieser Eigenschaften möglich.

Die Permeabilitätseigenschaften der Hülle können durch Poren in mindestens einer der Polyelektrolytschichten weiter modifiziert werden. Solche Poren können bei geeigneter Wahl durch die Polyelektrolyten selbst gebildet werden. Neben den Polyelektrolyten kann die Hülle aber auch andere Substanzen umfassen, um eine gewünschte Permeabilität zu erzielen. So kann insbesondere durch Einbringen von Nanopartikeln mit anionischen oder/und kationischen Gruppen oder von grenzflächenaktiven Substanzen, wie etwa Tensiden, die Permeabilität für polare Komponenten gesenkt werden. Durch die Inkorporation von selektiven Transportsystemen, wie z.B. Carriern oder Kanälen, in die Polyelektrolythülle, insbesondere in Lipidschichten, ist eine genaue Anpassung der transversalen Transporteigenschaften der Hülle an den jeweiligen Anwendungszweck möglich. Die Poren oder Kanäle der Hüllwand können durch chemische Modifizierung oder/und Änderung der Umgebungsbedingungen gezielt geöffnet bzw. verschlossen werden. So führt beispielsweise eine hohe Salzkonzentration des Umgebungsmediums zu einer hohen Durchlässigkeit der Hüllwand.

Die Modifizierung der Permeabilität von Polyelektrolythüllen kann durch Abscheidung von Lipidschichten oder/und amphiphiler Polyelektrolyten auf der Polyelektrolythülle nach Desintegration der Templatpartikel ist in EP 1 867 325 A1 beschrieben und nicht Gegenstand der vorliegenden Erfindung. Auf diese Weise kann die Permeabilität der Polyelektrolythüllen für kleine und polare Moleküle sehr stark vermindert werden. Beispiele für Lipide, die auf den Polyelektrolythüllen abgeschieden werden können, sind Lipide, die mindestens eine ionische oder ionisierbare Gruppe tragen, z.B. Phospholipide wie etwa Dipalmitoylphosphatidinsäure oder zwitterionische Phospholipide wie etwa Dipalmitoylphosphatidylcholin oder auch Fettsäuren bzw. entsprechende langkettige Alkylsulfonsäuren. Bei Verwendung zwitterionischer Lipide können Lipidmultischichten auf der Polyelektrolythülle abgeschieden werden. Auf den Lipidschichten können anschließend weitere Polyelektrolytschichten abgeschieden werden.

Die erfindungsgemäßen Kapseln weisen bevorzugt eine Hüllwanddicke von 2 bis 1000 nm, insbesondere 2 bis 100 nm, z.B. von 5 bis 8 nm, auf. Die Dicke der Hüllwand ist abhängig von der Anzahl der Schichten, der Polyelektrolythülle. Die Kapseln enthalten bevorzugt 2 bis 40, bevorzugt 2 bis 20, z.B. 3 bis 10 Schichten. Die Kapseln können jedoch auch eine größere Anzahl von Schichten, d.h. Polyelektrolytschichten und gegebenenfalls andere Schichten wie Lipidschichten, enthalten.

Die erfindungsgemäßen Kapseln zeichnen sich weiterhin durch ihre Monodispersität aus. So ist es möglich, eine Zusammensetzung mit einer Kapselverteilung zu erhalten, bei der der Anteil an Kapseln, deren Abweichung vom mittleren Durchmesser > 50% ist, weniger als 20%, bevorzugt weniger als 10% und besonders bevorzugt weniger als 1 %.

Die Kapseln sind sehr stabil gegenüber chemischen, biologischen, mechanischen und thermischen Belastungen. Die Kapseln können gegebenenfalls mit eingeschlossenen Wirkstoffen ohne Beeinträchtigung ihrer Eigenschaften getrocknet, eingefroren oder/und gefriergetrocknet werden. Nach dem Auftauen bzw. Resuspendieren in Wasser werden wieder intakte Kapseln erhalten.

Bei Trocknung oder Gefriertrocknung der Kapseln wird eine pulverförmige Zusammensetzung erhalten, die in geeigneten Lösungsmitteln, insbesondere in wäßrigen Lösungen resuspendiert werden kann. Ein weiterer Gegenstand der Erfindung ist deshalb eine getrocknete Kapseln umfassende Zusammensetzung. Die Trocknung kann nach bekannten Verfahren durchgeführt werden, insbesondere bei erhöhter oder verringerter Temperatur oder/und reduziertem Druck.

Das erfindungsgemäße Verfahren zur Herstellung von Kapseln mit einer Polyelektrolythülle, die mehrere Polyelektrolytschichten umfasst, und die einen Durchmesser bis zu 10 µm aufweisen, umfasst die Schritte:
a) Bereitstellen einer wässrigen Dispersion von Templatpartikeln geeigneter Größe,
b) Herstellen einer Hülle um die Partikel durch Aufbringen von Polyelektrolyten auf die Templatpartikel und
c) zumindest teilweise Desintegration der Templatpartikel.

Zunächst wird eine wäßrige Dispersion von Templatpartikeln geeigneter Größe bereitgestellt. Durch die Größe der Templatpartikel wird die Größe der Kapseln festgelegt. Anschließend werden mehrere Polyelektrolytschichten auf die Templatpartikel aufgebracht, wodurch ein umhülltes Templatpartikel gebildet wird. Die Form der Hülle hängt dabei unmittelbar von der Form der Templatpartikel ab.

Zum Aufbringen der Polyelektrolytschichten auf das Templat wird eine Dispersion der Templatpartikel in einer wässrigen Lösung erzeugt. Zu dieser Dispersion gibt man dann Polyelektrolytmoleküle, aus denen die erste Schicht aufgebaut werden soll. Diese Polyelektrolytmoleküle können die gleiche oder die entgegengesetzte Ladung wie die Oberfläche der Templatpartikel haben. Die Menge der zugegebenen Polyelektrolytmoleküle wird so gewählt, daß das gesamte Material für den Aufbau der ersten Schicht benötigt wird oder es wird ein Überschuß verwendet. Im letzteren Fall ist eine Entfernung der überschüssigen Polyelektrolytmoleküle, die nicht zum Aufbau der ersten Schicht benötigt werden, zweckmäßig, bevor die Zugabe von entgegengesetzt geladenen Polyelektrolytmolekülen für den Aufbau der zweiten Schicht erfolgt. Die Abtrennung der Polyelektrolytmoleküle kann nach bekannten Verfahren erfolgen, insbesondere Zentrifugation, Filtration oder/und Dialyse. Besonders bevorzugt ist die Abtrennung durch Membranfiltration wie im folgenden beschrieben. Anschließend werden weiterhin abwechselnd entgegengesetzt geladene Schichten von Polyelektrolytmolekülen aufgebracht, wobei für jede Schicht mit gleicher Ladung gleiche oder verschiedene Polyelektrolytspezies oder Gemische von Polyelektrolytspezies gewählt werden können. Die Anzahl von Schichten kann grundsätzlich beliebig gewählt werden. Neben Polyelektrolytmolekülen können auch andere Substanzen wie Nanopartikel, oberflächenaktive Substanzen oder/und Lipide auf den Templatpartikeln abgeschieden werden.

Als Templatpartikel können sowohl anorganische Materialien, z.B. Metalle, Keramiken, Oxide oder Salzkristalle als auch organische Materialien wie Polymerlatizes oder Melaminformaldehydpartikel, Lipidvesikel oder biologische Templatpartikel eingesetzt werden. Ebenfalls geeignet sind Emulsionströpfchen. Die Größe der Templatpartikel kann - insbesondere bei Verwendung biologischer Templatmaterialien - bis zu 10 µm erreichen. In den meisten Fällen ist die Größe der Templatpartikel jedoch besonders bevorzugt 5 nm bis 5 µm. Die Form der Templatpartikel ist nicht kritisch. Sowohl sphärische als auch anisotrope Partikel können beschichtet werden.

Es können auch Aggregate von Subpartikeln als Ausgangskerne (Templatpartikel) zur Beschichtung mit Polyelektrolyten eingesetzt werden. Diese Aggregate können gegebenenfalls in vorgeformtem oder präformiertem Zustand eingesetzt werden. Eine solche Präformierung kann beispielsweise durch Anlegen externer elektrischer Gleichstrom- oder/und Wechselfelder bzw. Magnetfelder auf Suspensionen mit Subpartikeln erzielt werden. Durch vorgeformte Aggregate kann die Form der Kapseln bestimmt werden. Weiterhin können solche Aggregate mit einer hohen Einheitlichkeit hinsichtlich der Größenverteilung erhalten werden (Monodispersität). Ebenso geeignet sind jedoch auch nichtvorgeformte Aggregate. Von besonderem Interesse sind Aggregate in sphärischer Form.

Die verwendeten Templatpartikel müssen nicht notwendigerweise geladen sein, um eine Selbstassemblierung von Polyelektrolytschichten zu ermöglichen. Vielmehr kann auf nichtgeladene Kerne ein geladener Precursorfilm aufgebracht werden, der an die Templatpartikel durch andere Wechselwirkungen, beispielsweise hydrophobe Wechselwirkungen, gebunden ist.

Nach Aufbringen der gewünschten Anzahl von Polyelektrolytschichten werden die umhüllten Templatpartikel zumindest teilweise desintegriert, insbesondere zerkleinert oder aufgelöst. Dabei verbleiben "leere" Kapseln mit einer Polyelektrolythülle. Die Auflösung der Templatpartikel wird unter Bedingungen durchgeführt, bei denen die Hüllen intakt bleiben. Eine Auflösung kann je nach Wahl des Templatpartikelmaterials z.B. thermisch oder chemisch erfolgen. Die bei der Auflösung entstehenden niedermolekularen Kernbestandteile können durch die Poren der Hülle nach außen gelangen. Auf diese Weise erhält man Kapseln mit Polyelektrolythüllen, die einen "leeren" Kern enthalten. Auf die leeren Polyelektrolytmolekülen können anderen Beschichtungssubstanzen aufgebracht werden.

Nach Desintegration der Templatpartikel kann im Inneren der Kapselhülle eine Flüssigphase vorliegen. Grundsätzlich können die Kapseln jede Flüssigkeit in ihrem Innern enthalten, z.B. eine wäßrige Flüssigkeit, insbesondere eine wäßrige Salzlösung oder Wasser, oder auch organische Lösungsmittel, insbesondere mit Wasser nicht mischbare Lösungsmittel wie Alkohole oder Kohlenwasserstoffe mit mindestens 4 C-Atomen. Weiterhin können die Kapseln in ihrem Inneren auch Feststoffe oder Gase enthalten. Bevorzugt werden teilvernetzte Melaminformaldehydpartikel als auflösbare Templatpartikel eingesetzt, die durch Einstellung des pH-Werts in dem die umhüllten Partikel enthaltenden Medium auf einen sauren Wert, z.B. ≤ 1,5, aufgelöst werden können, während die Hüllschicht selbst intakt bleibt. Die teilvernetzten Melaminformaldehydpartikel können auch durch chemische Reaktionen, insbesondere durch Sulfonierung in wäßrigen Medien aufgelöst werden. Als Sulfonierungsagentien werden bevorzugt Alkalisulfate, Alkalihydrogensulfite und andere wasserlösliche Salze der schwefeligen Säure verwendet. Weitere Beispiele für auflösbare Templatpartikel sind lösliche Polymerkerne, z.B. Harnstoff-Formaldehydpartikel, oder Salzkristalle.

Außerdem können als Templatmaterialien beispielsweise Zellen, z.B. eukaryontische Zellen, wie etwa Säugererythrozyten oder Pflanzenzellen, einzellige Organismen wie etwa Hefen, Bakterienzellen wie etwa E.coli Zellen, Zellaggregate, subzelluläre Partikel wie etwa Zellorganelle, Pollen, Membranpräparationen oder Zellkerne, Viruspartikel und Aggregate von Biomolekülen, z.B. Proteinaggregate wie etwa Immunkomplexe, kondensierte Nukleinsäuren, Ligand-Rezeptor-Komplexe etc. verwendet werden. Das erfindungsgemäße Verfahren eignet sich auch zur Verkapselung lebender biologischer Zellen und Organismen. Ebenso als Template geeignet sind Aggregate amphiphiler Materialien, insbesonder Membranstrukturen wie etwa Vesikel, z.B. Liposomen oder Micellen sowie andere Lipidaggregate.

Die Desintegration biologischer Templatpartikel kann durch Zugabe von Lysereagenzien erfolgen. Dabei sind Lysereagenzien geeignet, die biologische Materialien wie Proteine oder/und Lipide auflösen können. Vorzugsweise enthalten die Lysereagenzien ein Deproteinisierungsmittel, beispielsweise Peroxoverbindungen wie etwa H₂O₂ oder/und Hypochloritverbindungen wie etwa Natrium- oder Kaliumhypochlorit. Überraschenderweise erfolgt die Desintegration der Templatpartikel innerhalb einer kurzen Inkubationsdauer, z.B. 1 min bis 1 h bei Raumtemperatur. Die Desintegration der Templatpartikel ist weitgehend vollständig, da selbst bei elektronenmikroskopischer Betrachtung der verbleibenden Hüllen keine Reste der Partikel mehr nachweisbar sind. Bei Einbau biologischer Polyelektrolyte in die Hülle können leere Schichten auch innerhalb der Polyelektrolythülle erzeugt werden.

Die bei der Desintegration der Templatpartikel gebildeten Fragmente, z.B. im Fall von teilvernetzten Melaminformaledhypartikein, die bei Auflösung entstandenen Oligomere, können durch Poren, insbesondere Nanoporen, der Hüllwand aus dem Inneren der Kapseln nach außen austreten. Anschließend können sie - sofern gewünscht - von den Kapseln abgetrennt werden. Diese Abtrennung kann durch dem Fachmann bekannte Verfahren durchgeführt werden, z.B. durch Dialyse, Filtration oder/und Zentrifugation abgetrennt. Eine Abtrennung von Templatpartikelfragmenten ist oftmals jedoch nicht notwendig. Die Kapseln können auch ohne Abtrennungsschritt verwendet werden.

Die Beladung des Innenraums mit kleinen Molekülen kann dadurch erfolgen, daß die Permeabilität der Hülle als Funktion der externen physikalischen und chemischen Parameter variiert wird. Zur Beladung wird ein Zustand hoher Permeabilität eingestellt. Das eingeschlossene Material wird anschließend durch Veränderung der äußeren Parameter oder/und Verschluß der Poren, beispielsweise durch Kondensation der Hülle oder chemische Modifikation der Poren oder Kanäle zurückgehalten.

Die Polyelektrolythülle der Kapseln ist vorzugsweise für niedermolekulare Substanzen durchlässig, verhindert aber den Durchtritt von Makromolekülen. Damit stellt die Hüllwand eine Barriere gegenüber Mikroorganismen und von ihnen sekretierten externen Verdauungsenzymen dar. Deshalb können in die erfindungsgemäßen Kapseln biologisch abbaubare Substanzen eingeschlossen sein, ohne daß Konservierungsstoffe zur Haltbarmachung notwendig wären.

Die Kapseln können auch als Reaktionsräume für chemische Reaktionen oder als Präzipitations- oder Kristallisationstemplate verwendet werden, wobei man leere Kapseln oder einen Wirkstoff oder Katalysator enthaltende Kapseln einsetzen kann. Aufgrund der Tatsache, daß die Permeabilität der Kapselwände steuerbar ist, so daß sie beispielsweise niedermolekulare Substanzen passieren lassen, Makromoleküle jedoch weitgehend zurückhalten, lassen sich bei einer chemischen Reaktion entstehende hochmolekulare Produkte, z.B. bei einer Polymerisation entstehende Polymere auf einfache Weise während der Synthese im Innenraum zurückhalten. Das gleichzeitig im Außenmedium synthetisierte Reaktionsprodukt kann z.B. durch Zentrifugation oder/und Filtration nachträglich oder auch bereits während der Reaktion entfernt werden.

Während der Reaktion kann die Zufuhr des Reaktionssubstrats über die Diffusion durch die Kapselwände gesteuert werden. Dabei ergeben sich neue Wege, in Reaktionsabläufe einzugreifen. Da z.B. durch Filtration ein kontinuierlicher oder z.B. durch Zentrifugation auch ein plötzlicher Austausch des Außenmediums möglich ist, kann die Polymerisationsreaktion durch Substratentfernung beliebig angehalten werden bzw. das Monomer kann ausgetauscht werden. Es ist somit möglich, auf neue Art und Weise die Herstellung von definierten Co- oder Multipolymeren durchzuführen. Da durch die Permeation der Reaktionsablauf über die Monomerenzufuhr kontrollierbar ist, können in den Kapseln Produkte mit neuen und anderen Molekulargewichtsverteilungen, z.B. hoch monodisperse Produkte erzeugt werden. Im Kapselinneren synthetisierte Polymere lassen sich z.B. durch NMR, durch IR, spektroskopisch durch Titration mit Fluoreszenzfarbstoffen und durch konfokale Mikroskopie nachweisen. Mit Einzelteilchenlichtstreuung läßt sich der Massenzuwachs und somit die Reaktionskinetik verfolgen.
Bei Verwendung anisotroper Kapseln zur Verpackung von Wirkstoffen oder als Reaktionsräume, z.B. für Synthesen oder Präzipitationsprozesse, und gegebenenfalls anschließender Auflösung der Templathüllen können Partikelzusammensetzungen als Dispersionen mit vorbestimmten Formen und Gestalten erzeugt werden. Die Erfindung betrifft somit auch anisotrope Partikelzusammensetzungen, die durch Verkapselung von Wirkstoffen in einer Polyelektrolythülle, z.B. durch Synthese oder Präzipitation und anschließende Entfernung des Templats, z.B. durch thermische oder chemische Behandlung, erhältlich sind. Vorzugsweise besitzen diese anisotropen Partikel die Form der als Templat verwendeten Strukturen. Anisotrope Partikel können bei Anlagen von Felder bewegt, z.B. gedreht oder ausgerichtet werden. Auf diese Weise können Dispersionen mit schaltenden Eigenschaften erzeugt werden.

Weiterhin können die Kapseln zum Einbringen von organischen Flüssigkeiten wie etwa Alkoholen oder Kohlenwasserstoffen, z.B. Hexanol, Octanol, Octan oder Decan, oder zum Verkapseln von Gasen verwendet werden. Solche mit einer organischen, nicht mit Wasser mischbaren Flüssigkeit gefüllten Kapseln können auch für chemische Reaktionen, z.B. Poly-merisationsreaktionen eingesetzt werden. So kann das Monomer über dessen Verteilungsgleichgewicht gezielt im Innenraum der Kapseln angereichert werden. Gegebenenfalls kann die Monomerenlösung bereits vor Beginn der Synthese im Innenraum eingekapselt werden.

Die Kapseln können auch auf einer Oberfläche immobilisiert werden. Die Einstellung der Ladung der äußeren Schicht und die freie Funktionalisierbarkeit der Außenhülle erlaubt eine vom Zustand der eingeschlossenen Moleküle unabhängige Immobilisierung der Kapseln. Dies eröffnet zahlreiche Anwendungsmöglichkeiten, vor allem im Bereich der Sensorik und Oberflächenanalytik. Dabei können die mit Polyelektrolyt beschichteten Templatpartikel an eine Oberfläche adheriert und die Templatpartikel dann aus den bereits immobilisierten beschichteten Kernen herausgelöst werden, um immobilisierte Kapseln zu bilden. Ebenso kann jedoch die Auflösung der Kerne vor einer Deponierung an der Oberfläche erfolgen.

Die Kapseln können auf zahlreichen Anwendungsgebieten, beispielsweise Sensorik, Oberflächenanalytik, als Emulsionsträger, Mikroreaktionsräume wie etwa für katalytische Prozesse, Polymerisation-, Präzipitations- oder Kristallisationsprozesse, in der Pharmazie und Medizin, z.B. zum Targeting von Wirkstoffen oder als Ultraschallkontrastmittel, in der Lebensmitteltechnologie, Kosmetik, Biotechnologie, Sensorik, Informationstechnologie, Druckindustrie (Einkapselung von Farbstoffen), photographische Industrie eingesetzt werden. Weiterhin können die Kapseln zum Aufbau von Mikro- bzw. Nanokompositen, d.h. Werkstoffen, die aus mindestens zwei verschiedenen Materialien bestehen und eine mikro- bzw. nanoskopische Ordnung aufweisen, eingesetzt werden.

Bei der Verwendung der Kapseln als Reaktionsräume können niedermolekulare Substanzen, wie z.B. Edukte und Produkte, durch die Hüllwände permeiren, während beispielsweise die Katalysatoren eingeschlossen sind. Bei der Verwendung von mit Katalysatoren beladenen Mikro- bzw. Nanokapseln, wobei die Kapseln beispielsweise in einer Säule gepackt sind, steht für die Reaktion beträchtlich mehr Katalysator zur Verfügung, als bei herkömmlichen oberflächengebundenen Katalysatoren, da dort die Größe der Oberfläche limitierend ist. Besonders vorteilhaft ist, daß der Katalysator im Kapselinneren von der Produktion nicht durch aufwendige Verfahren wieder abgetrennt werden muß. Weiterhin ist die Lebensdauer der Katalysatoren verbessert, da makromolekulare Stoffe, insbesondere Bakterien und Pilze, nicht durch die Hüllwände gelangen können. Dadurch werden die an viele Verfahren gestellten hohen Anforderungen an die Sterilität verringert, was viele, technisch einfache Anwendungen von biologischen Katalysatoren eröffnet.

Auch Sensormoleküle können in die Kapseln eingeschlossen werden. Dabei kann es sich um Enzyme handeln, die in Anwesenheit eines Substrats unter geeigneten Bedingungen optisch oder auf andere Weise nachweisbare Produkte, beispielsweise farbige oder fluoreszierende Produkte bilden. Es können aber auch elektrisch aktive Sensormoleküle, insbesondere oxidierbare oder reduzierbare Stoffe, eingeschlossen werden, wobei die Kapseln auf Elektroden immobilisiert werden können. Hier ist neben der schützenden Funktion der Kapseln insbesondere von Vorteil, daß das Sensormolekül, nicht direkt mit der Elektrode in Berührung kommt.

Die Kapseln können auch zur Herstellung von Kristallen oder amorphen Präzipitaten aus organischen oder anorganischen Materialien oder zum Einschluß von organischen oder anorganischen Kristallen oder amorphen Präzipitaten verwendet werden. Bevorzugt dienen die Kapseln als Kristallisations- oder Präzipitationsraum bzw. Template zur Herstellung von insbesondere monodispersen Kristallen oder Präzipitaten. Mit den erfindungsgemäßen Kapseln ist ein hoher Grad an Monodispersität zu erhalten, da die maximale Größe der eingeschlossenen Partikel durch die Größe der Kapseln begrenzt ist. Als Kristallisationskeime können chemische Gruppen an der inneren Hüllwand verwendet werden. Dazu werden beim schichtweisen Aufbau der Hülle der Kapseln in der innersten Schicht Moleküle verwendet, die Seitenketten aufweisen, die das Kristallwachstum begünstigen. So können beispielsweise Polyphosphate an der Innenseite der Hülle aufgebracht werden, um im Inneren CaCO₃ zu bilden. Als äußerste Schicht der Polyelektrolythülle der Kapseln werden günstigerweise Polyelektrolyte verwendet, die ein Kristallwachstum unterdrücken, beispielsweise Amine.

Die Kapseln können auch zum Aufbau von Mikro- bzw. Nanokompositen verwendet werden. Mikro- und Nanokomposite sind Werkstoffe, die aus mindestens zwei verschiedenen Materialien bestehen und eine mikro- bzw. nanoskopische Ordnung aufweisen. Solche Komposite imitieren oftmals in der Natur vorliegende Produkte, wie z.B. Muschelschalen, die aus Nanokompositen von gewöhnlichen Kalk- und Eiweißmolekülen bestehen. Solche Komposite haben bei geringem Gewicht eine überraschend hohe Festigkeit. Durch die Assemblierung können makroskopische Strukturen geordnet aufgebaut werden.

Anisotrope Hüllen, die unter Verwendung anisotroper Templatpartikel, z.B. biologischer Templatpartikel hergestellt wurden, erlauben in Verbindung mit z.B. Kristallisation oder/und Präzipitation die Herstellung von Kompositen mit anisotropen Eigenschaften. So können beispielsweise magnetische Ellipsoide hergestellt werden, z.B. durch Füllung mit magnetischen Partikeln oder/und durch Adsorption von magnetischen Nanopartikeln auf der Polyelektrolythülle. Im Magnetfeld zeigen diese anisotropen Partikel eine Orientierung, wodurch die optischen Eigenschaften einer Partikelsuspension rasant geändert werden können (magneto-optischer Schalter). Auf analoge Weise kann man mit ferroelektrischen Partikeln verfahren. Mit Hilfe dieser Teilchen kann man beispielsweise mit einem rotierenden Feld kleine Schaufelräder zum Pumpen erzeugen (Mikromechanik). Daneben können anisotrope Partikel durch Dissipation erwärmt werden. Dies kann zur Erzeugung extrem lokalisierter Wärmequellen genutzt werden, die man mit elektrischen bzw. mit magnetischen Feldern bewegen kann. Dies erlaubt die Erzeugung lokaler Hyperthermieffekte. Weiterhin können durch geordnete Ausrichtung anisotroper Partikel Kompositmaterialien mit hierarchischer Struktur und interessanten makroskopischen physikalischen anisotropen Eigenschaften erzeugt werden.

Wie bereits ausgeführt, kann die Permeabilität der Polyelektrolythülle durch Modifikationen, z.B. Aufbringen von Lipidschichten, gesteuert werden. Dies kann für pharmazeutischen Anwendungen genutzt werden, indem nach der Verkapselung polarer niedermolekularer Substanzen Lipide auf die Hülle aufgetragen werden, um auf diese Weise die Permeabilität der Hülle für die verkapselten Substanzen zu vermindern. Der verkapselte Stoff kann nur noch langsam durch die Lipidschicht mit einer über lange Zeit konstanten Rate austreten, was für pharmakologische Applikationen oftmals wünschenswert ist.

Durch die Verkapselung und anschließende Auflösung von Templaten können formgetreue dreidimensionale Abdrücke von Templatpartikeln hergestellt werden. Bei Blockvemetzung der Polyelektrolythüllen erhält man mesoporöse Materialien mit monodisperser formgetreuer Porenverteilung. Diese Materialien besitzen eine hohe innere Oberfläche verbunden mit hoher Festigkeit und machen sie zu hervorragenden Filtersubstanzen für industrielle Zwecke. Durch Auswahl der Template (Form und Größe) können mesoporöse Materialien mit vorgegebenen Poren hergestellt werden.

Natürlich kann durch Variation der für die Herstellung der Polyelektrolythülle verwendeten Materialien auch die Oberflächenchemie in weiten Bereichen variiert werden.

Schließlich können die Polyelektrolythüllen auch zur Erzeugung von pH-Gradienten zwischen dem inneren der Hülle und dem die Hülle umgebenden Volumen verwendet werden.

Die Erfindung wird durch die beigefügten Figuren und Beispiele weiter erläutert.
- Fig.1: stellt eine schematische Veranschaulichung einer bevorzugten Ausführungsform des erfindungsgemäßen Verfahrens dar.
- Fig.2: zeigt die Schichtdicke als Funktion der Zahl der Schichten bei der Absorption von Poly(allylaminhydrochlorid) (PAH) und Poly(styrolsulfonat, Natriumsalz) (PSS) auf negativ geladene Polystyrol (PS)-Latexpartikel.
- Fig.3: zeigt ein SEM-Abbild (Rasterelektronenmikroskopie) einer Polyelektrolythülle mit neun Schichten (PSS/PAH)₄/PSS nach Auflösung des Kerns. Die äußere Schicht ist PSS.
- Fig.4: zeigt ein TEM-Abbild (Transmissionselektronenmikroskopie) einer Polyelektrolythülle mit neun Schichten (PSS/PAH)₄/PSS.
- Fig.5: zeigt atomkraftmikroskopische Abbildungen von PSS/PAH- Polyelektrolythüllen. Die Zahl der Polyelektrolythüllen beträgt in Fig. 5(A) 3 PSS/PAH/PSS und in Fig.5(B) 9 (PSS/PAH)₄/PSS].

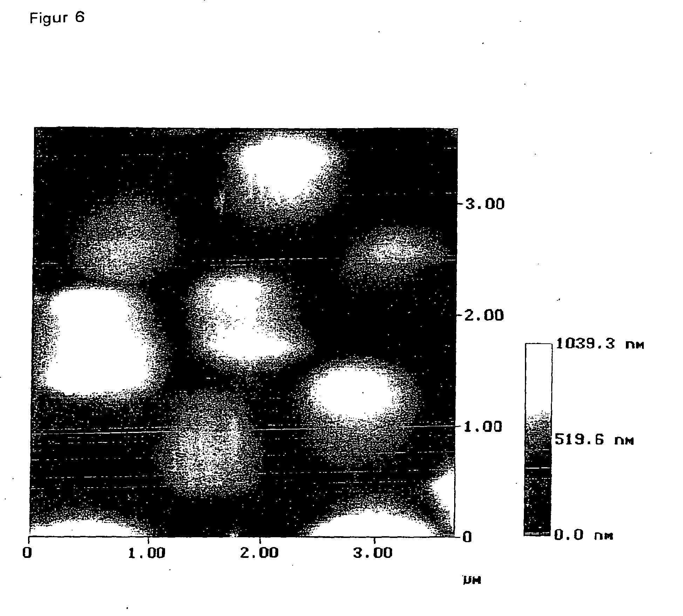

- Fig.6: zeigt ein AFM-Abbild von PS-Latexteilchen mit 1,28 µm Durchmesser und einer Polyelektrolythülle mit sechs Schichten (PAH/PSS)₃. Die äußere Schicht ist PSS.

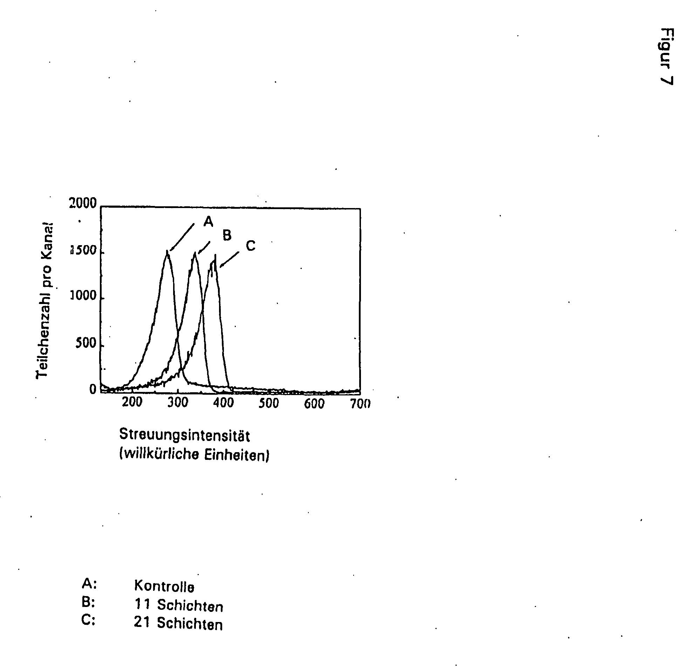

- Fig.7: zeigt normalisierte Lichtstreuungs-Intensitätsverteilungen von PAH/PSS-beschichteten PS-Latexpartikeln. Partikel mit 11 und 21 Schichten werden mit den unbeschichteten Partikeln verglichen.

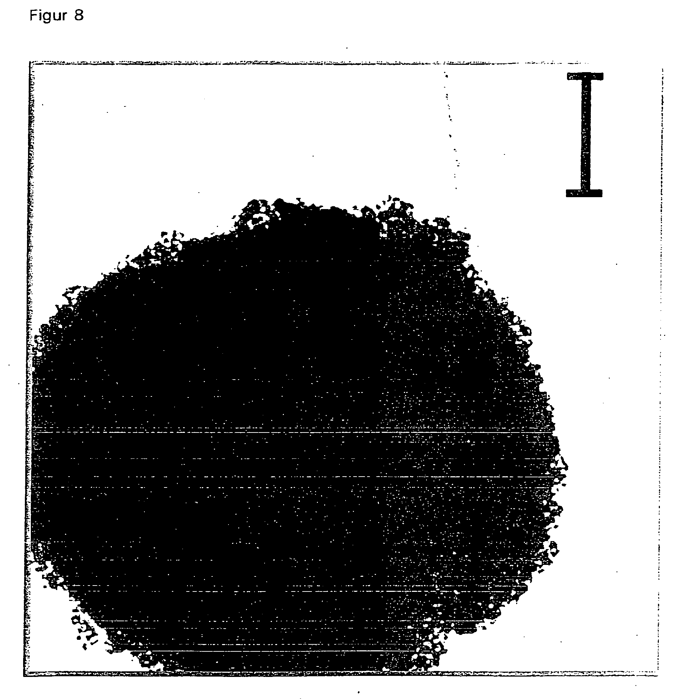

- Fig.8: zeigt ein TEM-Abbild eines PS-Latexpartikels beschichtet mit vier Schichten PAH/PSS und anschließend drei Schichtpaaren bestehend jeweils aus einer Schicht Magnetit und einer Schicht PSS. Die Skala entspricht 200 nm.

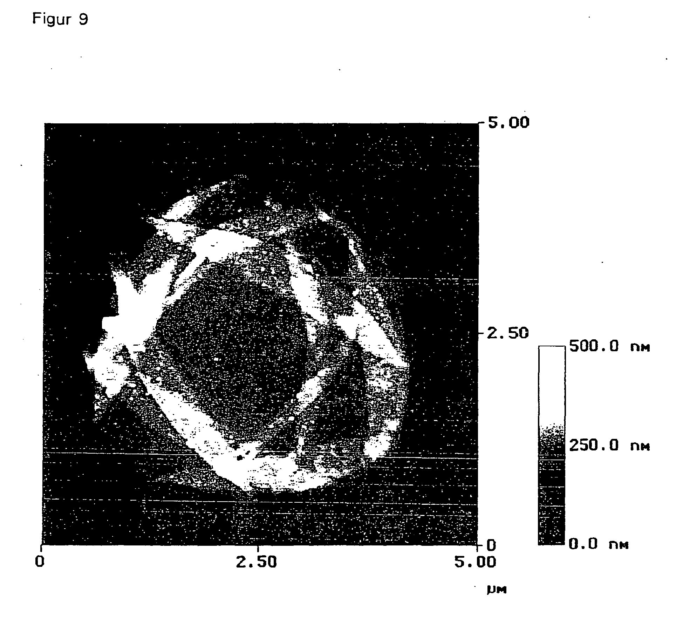

- Fig. 9: zeigt ein AFM-Abbild einer Polyelektrolythülle erzeugt durch Beschichtung eines 3,7 µm MF-Latexpartikels mit 10 Schichten PSS/ PAH und anschließende Auflösung des Templatpartikels.

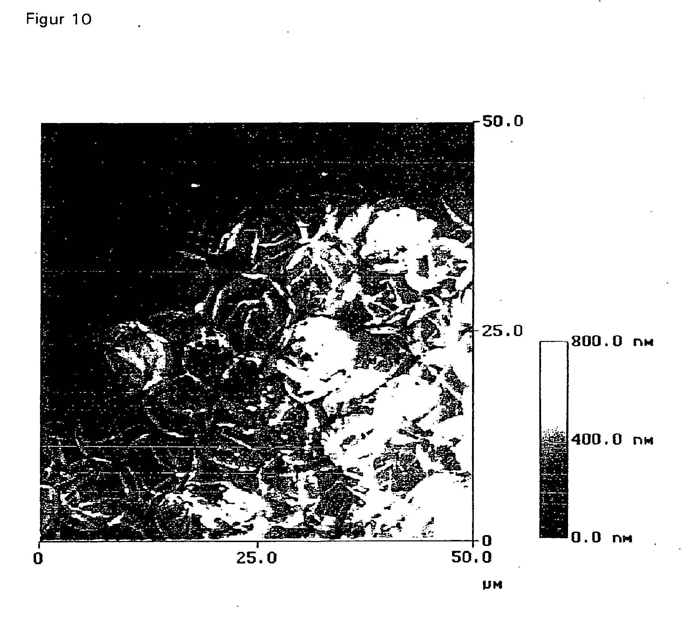

- Fig.10: zeigt ein AFM-Abbild von Polyelektrolythüllen erhalten durch Beschichtung von Glutardialdehyd-fixierten humanen Erythrozyten mit zehn Schichten PSS/PAH und anschließende Auflösung des Templatpartikels. Die Skalenwerte der Achsen sind in µm und die Werte der Höhenskala in nm angegeben.

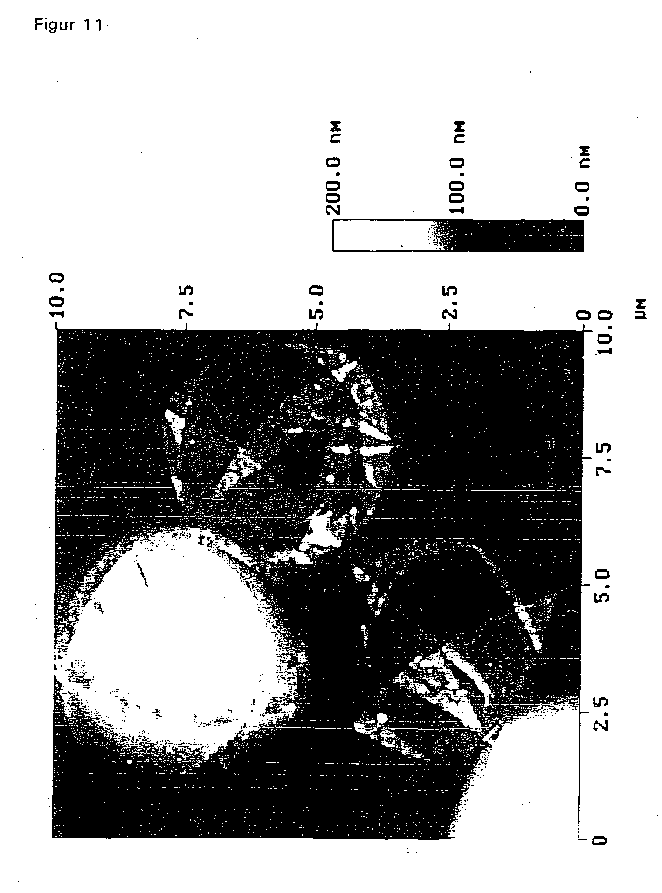

- Fig.11: zeigt ein AFM-Abbild von Polyelektrolythüllen erhalten durch Beschichtung von 3,7 µm MF-Latexpartikeln mit zehn Schichten Chitosan/Chitosansulfat und anschließende Auflösung des Templatpartikels.

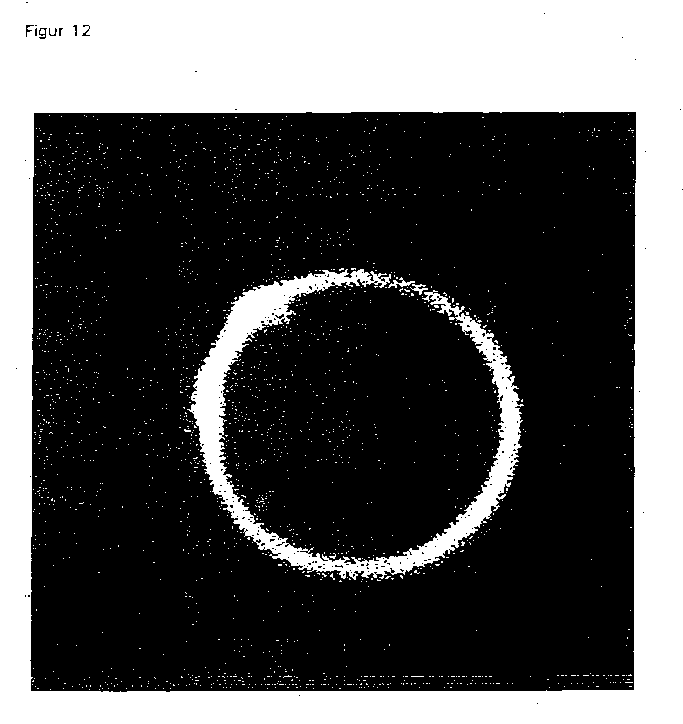

- Fig.12: zeigt ein CLSM-Abbildung von Chitosan/Chitosansulfatmikrokapseln bestehend aus elf Schichten, wobei als äußerste Schicht FITC-markiertes PAH verwendet wurde.

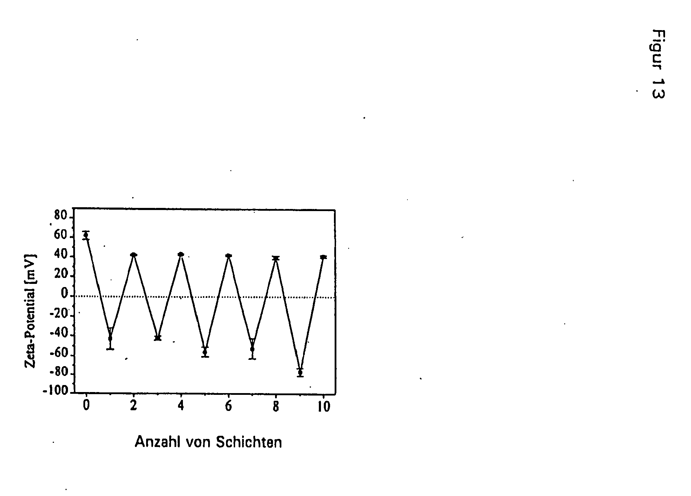

- Fig.13: zeigt das Zetapotential von beschichteten und mit Chitosan/Chitosansulfat beschichteten 3,7 µm MF- Latexpartikeln.
- Fig.14: zeigt die Fluoreszenzintensität einer 6-CF als Fluoreszenzmarker enthaltenden Suspension von Polyelektrolythüllen gegenüber dem pH-Wert des umgebenden Mediums die durch HCl oder H-PSS mit dem jeweils angegebenen Molekulargewicht titriert wurde.
- Fig.15: zeigt die Beziehung des pH-Werts im Inneren der Kapseln zum pH-Wert des umgebenden Mediums, titriert mit H-PSS (MW 4200) in Abwesenheit von Salz (ausgefüllte Kreise) und in Gegenwart von 1 mM NaCl (leere Kreise). Die gestrichelte Linie zeigt die durch Titration der Kapseldispersion mit HCl erhaltene Kontrolle.

### Beispiele

### Beispiel 1 Herstellung von teilvernetzten Melaminformaldehyd-Templat-partikeln

Monodisperse Melaminformaldehydpolymerpartikel können durch eine Polykondensationsreaktion aus Melaminformaldehydpräkondensaten im Größenbereich bis zu 15 µm hergestellt werden (vgl. DD 224 602). Die Größe der Partikel kann durch die Monomerkondensation, den pH-Wert, die Reaktionstemperatur und den Tensidzusatz beeinflußt werden. Bei den im Stand der Technik beschriebenen Verfahren werden hochvernetzte Partikel erhalten, die in den meisten organischen Lösungsmitteln, wie etwa Xylol, Toluol und Alkohol sowie in Säuren und Basen unlöslich sind. Zur Herstellung von auflösbaren, teilvernetzten Melaminformaldehyd-Templat-Partikeln wird das im Stand der Technik beschriebene Verfahren modifiziert, indem der Polykondensationsprozeß auf einer bestimmten Initialstufe der Reaktion unterbrochen wird. Dadurch werden in wäßrigen Medien lösliche Kerne erhalten. Der Abbruch der Reaktion kann durch rasche Temperaturabsenkung, durch Veränderung des pH-Werts in den alkalischen Bereich und durch Wahl geeigneter Präkondensate, insbesondere Tetramethylolmelamin, erreicht werden.

Die so erhaltenen Kerne können durch Säurezugabe und/oder durch bestimmte chemische Reaktionen, insbesondere Sulfonierung in wäßrigen Medien, aufgelöst werden. Als Sulfonierungsagentien können insbesondere Alkalisulfite, Alkalihydrogensulfite und andere wasserlösliche Salze der schwefligen Säure eingesetzt werden. Die Auflösbarkeit der Kerne kann durch den Zeitpunkt der Unterbrechung des Polykondensationsprozesses beeinflußt werden. Die Unterbrechung wird 1 min bis 3 h nach Start der Reaktion in Abhängigkeit der Reaktionsbedingungen und in Abhängigkeit der gewünschten Auflösbarkeit der Kerne durchgeführt. Die Auflösungsgeschwindigkeit kann weiterhin durch die Wahl von pH-Wert, Temperatur und Sulfonierungsreagens gesteuert werden. Somit ist es möglich, Kerne mit einer Auflösegeschwindigkeit von 0,1 s bis 10 h, wiederum in Abhängigkeit der Auflösungsbedingungen, zu erhalten. Diese auflösbaren Melaminformaldehydpartikel werden hierin als teilvernetzte Melaminformaldehydpartikel bezeichnet.

### Beispiel 2 Herstellung von leeren Polyelektrolythüllen unter Verwendung von Melaninformaldehydpartikeln als Templat

### 2.1

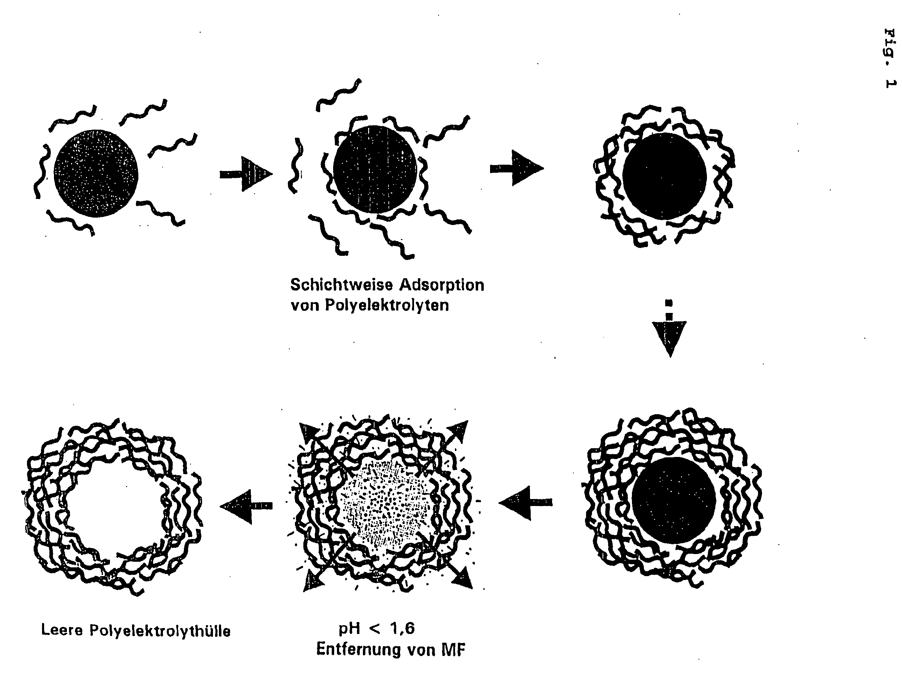

Auf wie in Beispiel 1 beschrieben hergestellte monodisperse, kolloidale, teilvernetzte Melaminformaldehydpartikel (MF) mit einem Durchmesser von 2,0 bzw. 3,3 µm werden schrittweise Polyelektrolyte aus verdünnten wäßrigen Lösungen aufgebracht (vgl.Fig.1). Die Polyelektrolytschichten werden durch alternierende Adsorption von entgegengesetzt geladenen Polyionen, beginnend mit der Adsorption eines negativ geladenen Polyanions (z.B. Polystyrolsulfonat, Natriumsalz; PSS) auf die positiv geladenen MF-Partikel aufgebracht. Typische Adsorptionsbedingungen waren 20 mM Polyelektrolyt (Konzentrationsangabe bezogen auf Monomer) 0,5 M NaCl bei Partikelkonzentrationen von 0,5 Gew.%. Die Adsorptionsdauer betrug 20 min. Die MF-Partikel hatten eine Dichte von 1,5 g/cm³.

Nach Beendigung dieses Adsorptionszyktus wurde überschüssiger Elektrolyt durch wiederholte Zentrifugations/Waschzyklen entfernt. Dazu wurden die beschichteten Kerne bei einer Zentrifugationsgeschwindigkeit von 2000 U/min (unter Verwendung eines Eppendorf-Rotors) abgesetzt. Dann wurden drei Waschschritte mit entionisiertem Wasser vor der Zugabe des nächsten Polyelektrolyten durchgeführt, um die vollständige Entfernung von nichtadsorbiertem Polyelektrolyten sicherzustellen. Durch Wiederholung dieser Vorgehensweise kann die gewünschte Zahl von Polyelektrolytschichten aufgebracht werden.
Anschließend wurde der pH-Wert auf < 1,6 erniedrigt, wodurch die MF-Kerne innerhalb weniger Sekunden aufgelöst werden. Die Fragmente dringen durch die Poren der Hülle nach außen und können entfernt werden, so daß eine leere Polyelektrolythülle erhalten wird. Bei pH-Werten oberhalb 1,8 wird keine nachweisbare Auflösung der Kerne beobachtet.

### 2.2

100 µl einer 3%-igen Dispersion von teilvernetzten MelaminformaldehydPartikeln mit einer Partikelgröße von 3 µm werden mit 400 µl einer Lösung von 20 mono mM PSS in 0,5 M NaCl versetzt. Nach einer Einwirkzeit von 5 min unter leichtem Schütteln wird 1 ml reines Wasser zugesetzt. Nach Zentrifugation bei 2000 U/min wird der Überstand decantiert, das Sediment mit reinem Wasser aufgefüllt und die Zentrifugation wiederholt. Abermaliges Decantieren und ein weiterer Zentrifugationszyklus ergeben gereinigte, mit einer PSS-Schicht bedeckte MF-Partikel. In analoger Weise wird anschließend eine Poly(allylaminhydrochlorid)-Schicht (PAH) aufgebracht. Diese Zyklen werden abwechselnd in Abhängigkeit von der gewünschten Zahl der Schichten wiederholt. Nach dem Zentrifugationszyklus am Ende des Aufbaus der letzten Schicht werden 1 ml einer 0,1 N Salzsäure zugegeben. Nach etwa 5 min Schütteln erhält man eine klare Lösung, da die durch die Partikel verursachte Trübung der Lösung verschwunden ist. Anschließend zentrifugiert man für etwa 10 min bei 10 000 U/min. Bei dieser Zentrifugation scheidet sich ein feiner, leicht milchig wirkender Bodensatz ab, der die gebildeten Polyelektrolythüllen enthält. Bereits ein leichtes Schütteln nach Zusatz von Wasser ist ausreichend um die Hüllen zu resuspendieren. Nach zwei weiteren Zentrifugationsschritten erhält man eine gereinigte, 3%-ige Dispersion von sphärischen, monodispersen Polyelektrolythüllen in Wasser. Eine Probe dieser Hüllen kann durch Rasterelektronenmikroskopie, Transmissionselektronenmikroskopie und/oder Atomkraftmikroskopie untersucht werden.

### 2.3

1,59 mg Na-Polystyrolsulfonat (PSS) werden einer Dispersion von teilvernetzten Melaminformaldehydpartikeln in 0,5 M NaCl zugesetzt. Die MF-Dispersion enthält insgesamt 2,2 x 10⁸ Partikel. Nach zwanzigminütigem leichtem Schütteln werden 0,81 mg Polyallylaminhydrochlorid zugesetzt. Nach wiederum 20 min werden unter leichtem Schütteln 1,59 mg PSS zugegeben. Diese Vorgehensweise wird 5x mit jeweils einer PAH- und einer PSS-Zugabe wiederholt. Man erhält dadurch Melaminformaldehydpartikel, die mit 13 alternierenden Schichten bedeckt sind. Durch Zugabe von 10 ml 1 N-Salzsäure wird der pH-Wert erniedrigt, so daß sich die MF-Kerne auflösen. Durch die Zentrifugation bei 15 000 g für 15 min werden die Polyelektrolythüllen vom Überstand abgetrennt.

### Beispiel 3 Charakterisierung der Polyelektrolythüllen

### 3.1 Abhängigkeit der Schichtdicke von der Anzahl der Schichten

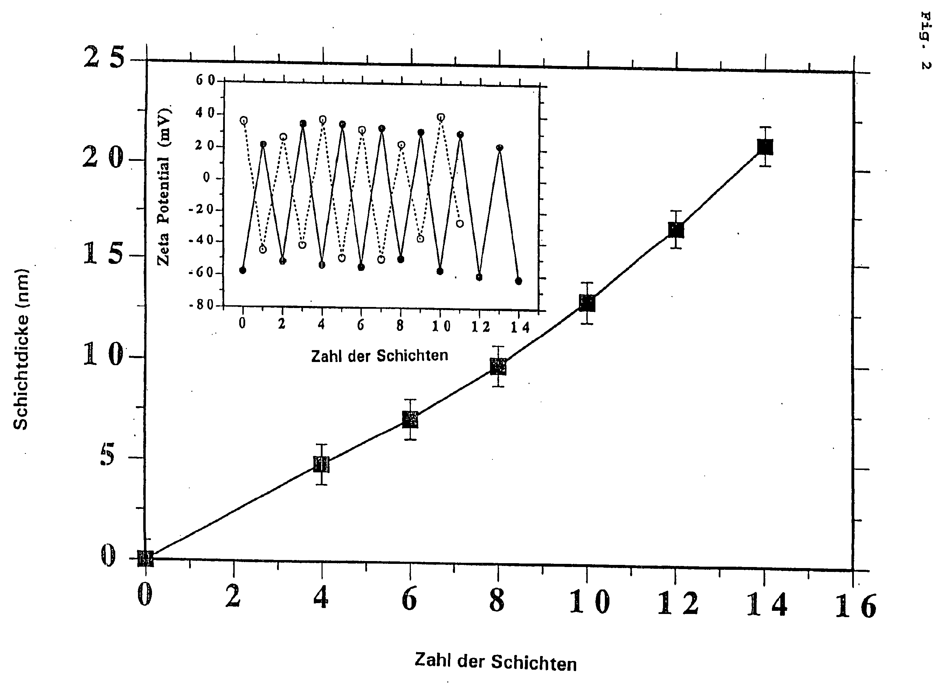

Auf negativ geladene Polystyrol (PS)-Latexpartikel wurden abwechselnd Schichten aus Poly(allylaminhydrochlorid) (PAH) und Poly(styrolsulfonat, Natriumsalz) (PSS) adorbiert. Die Schichtdicke wurde durch EinzelteilchenLichtstreuung gemessen. Der Intensitätsanstieg des gestreuten Lichts ist ein Maß für die adsorbierte Menge und wurde in die Schichtdicke unter Verwendung des Refraktionsindex der Polyeletrolytschichten konvertiert. Das Insert in Fig.2 gibt das aus elektrophoretischen Mobilitätsmessungen (Malvern Zetasizer 4) abgeleitete Zetapotential für die Adsorption von PAH und PSS auf Polystyrolpartikel (ausgefüllte Kreise) und für die Adsorption von PSS und PAH auf positiv geladene MF-Partikel (offene Kreise) an.

Das Zetapotential ist ein Maß für die effektive Ladungsdichte auf der Partikeloberfläche. Wie aus Fig.2 ersichtlich ist, tritt eine Umkehrung des Oberflächen potentials mit der Adsorption jeder Polyelektrolytschicht auf die Polystyrol- bzw. MF-Partikel auf. Eine Umkehrung des Oberflächenpotentials begünstigt die anschließende Adsorption des entgegengesetzt geladenen Polyions.

Flugzeitmassenspektrometrische Untersuchungen haben gezeigt, daß bei der Auflösung der teilvernetzten MF-Templatpartikel bei einem pH-Wert < 1,6 MF-Oligomere gebildet werden, die hauptsächlich aus 5-10 Einheiten Tetramethylolmelamin bestehen. Diese MF-Oligomere haben eine charakteristische Querschnittsausdehnung von etwa 1 nm, wie durch molekulare Dynamiksimulationen bestimmt (unter Verwendung des Programms DISCOVERY). Diese Oligomere werden aus dem Kern ausgestoßen und permeiren durch Polyelektrolytschichten, die die Hülle bilden, und können schließlich von den leeren Schalen durch Zentrifugation abgetrennt werden. Dies bestätigt, daß die Hüllen für Moleküle mit einer Größe im Bereich von wenigen nm, insbesondere ≤ 10 nm, bevorzugt ≤ 5 nm, leicht permeabel sind.

### 3.2 Rasterelektronenmikroskopische Untersuchungen der Polyelektrolythüllen

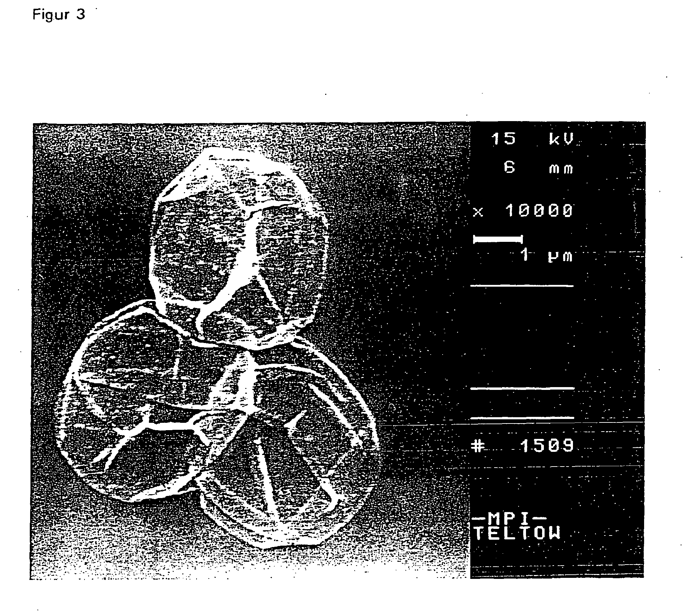

Die Polyelektrolythüllen wurden mittels Rasterelektronenmikroskopie (SEM) untersucht. Zunächst wurde ein MF-Kern mit einem Durchmesser von 3,3 µm mit 9 Polyelektrolytschalen (PSS/PAH)₄/PSS beschichtet. Die äußerste Schicht ist PSS. Nach Auflösung des MF-Kems wurden die erhaltenen Kapseln mit SEM untersucht. Wie aus Fig.3 ersichtlich, liegen die Durchmesser im Bereich von 4,0 ± 0,5 µm. Die Schalen werden durch eine starke elektrostatische Anziehung an die positiv geladene, Poly(ethylenimin)-beschichtete Glasoberfläche immobilisiert. Weiterhin tritt während der Untersuchung ein gewisses Ausmaß an Austrocknen der Kapseln ein. Dies führt dazu, daß die Hülle faltig wird. Wie jedoch aus Fig.3 ersichtlich ist, sind keine Löcher oder Rißspuren in den Hüllen zu finden.
Die SEM-Messungen wurden unter Verwendung eines Zeiß-DSM40-Instruments durchgeführt, welches bei einer Beschleunigungsspannung von 15 KeV betrieben wurde. Die Proben wurden durch Aufbringen eines Tropfens einer die Hüllen enthaltenden Lösung auf Poly(ethylenimin)-beschichtetes Glas hergestellt. Nachdem sich die Hüllen auf den Glasträgern abgesetzt hatten, wurden sie gründlich mit Wasser gespült und vorsichtig unter einem Stickstoffstrom getrocknet.

### 3.3 Transmissionselektronenmikroskopie (TEM)

Auf MF-Templatpartikel mit einem Durchmesser von 2 µm wurden neun Schichten Polyelektrolyt (PSS/PAH)₄/PSS aufgebracht. Anschließend wurden die Templatpartikel aufgelöst. Die Proben wurden mit Glutardialdehyd, OsO₄ und K₂Cr₂O₇ fixiert, in Ethanol/Aceton dehydriert, in ein Epon 812/Araldit M-Harz eingebettet und in einem Ofen für zwei Tage polymerisiert. Dünnschnitte (80 bis 100 nm) wurden unter Verwendung eines Reichert-Ultratom angefertigt und mit Uranylacetat und Bleicitrat eingefärbt. Die Messungen wurden auf einem JEOL 100 B Elektronenmikroskop durchgeführt.

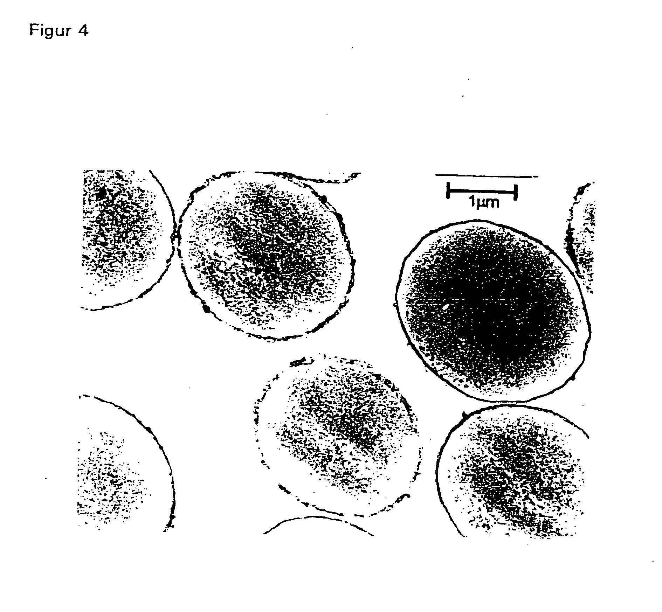

Wie aus Fig.4 ersichtlich, kann die angefärbte Polyelektrolytschicht, die das weniger angefärbte Zellinnnere umgibt, deutlich identifiziert werden. Die homogene Form der Hüllen zeigt, daß die hergestellten Kapseln sowohl den Durchmesser als auch die sphärische Form der Templatpartikel beibehalten, unter der Voraussetzung, daß die innere wäßrige Lösung nicht entfernt wird. Weiterhin ist zu erkennen, daß die Dicke der aus neun Schichten bestehenden Polyelektrolythülle in der Größenordnung von 20 nm ist. Dieser Wert stimmt mit den in Fig.2 gezeigten Daten für Polyelektrolyt-beschichtete Polystyrolpartikel überein. Daraus kann man schließen, daß die Art der Templatpartikel die Dicke der Polyelektrolytschichten nicht wesentlich beeinflußt. Aus dem TEM-Abbild ist auch ersichtlich, daß die Polyelektrolythüllen weder Risse noch Löcher aufweisen.

### 3.4 Atomkraftmikroskopische (AFM) Untersuchungen

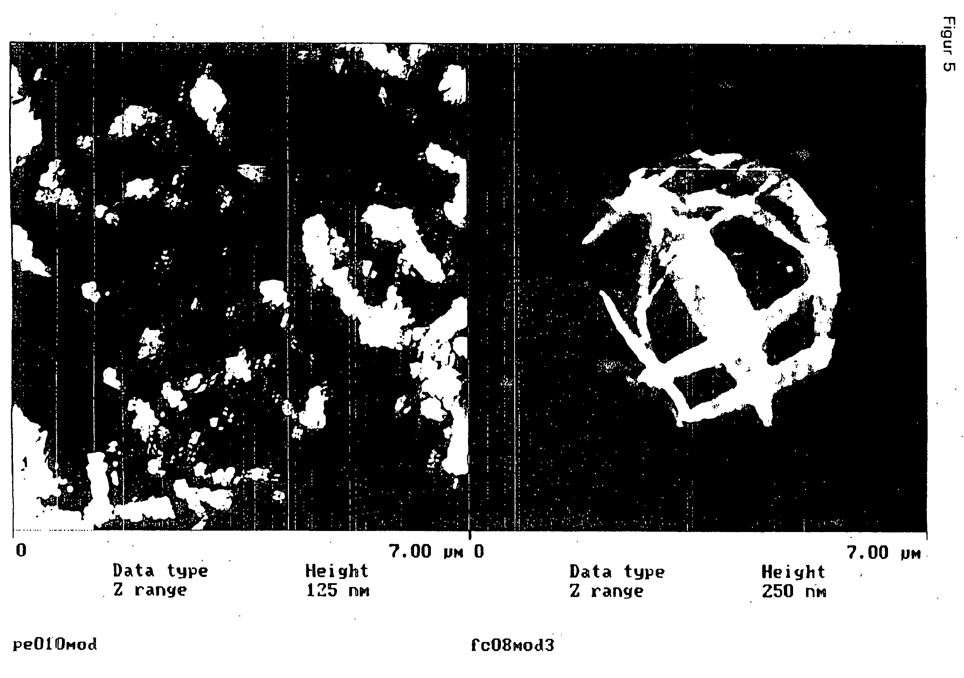

PSS/PAH-Polyelektrolythüllen wurden unter Verwendung von MF-Templat-partikeln mit einem Durchmesser von 3,3 µm wie oben beschrieben hergestellt. Die Zahl der Polyelektrolytschichten betrug 3 PSS/PAH/PSS (Fig.5(A)) bzw. 9 (PSS/PAH)₄/PSS (Fig.5(B)). Diese Kapseln wurden mit AFM im Tapping Mode (TM) untersucht. Fig.5 zeigt, daß die dreidimensionalen Polyelektrolythüllen durchgehende Folien sind, die Falten aufweisen, die von der Verdampfung des wäßrigen Inneren resultieren. Wie zu sehen ist, steigt die Höhe der Kapseln mit ansteigender Schichtzahl an. Die maximale Höhe der getrockneten Hüllen in Abbildung A liegt in der Größenordnung von 50 nm und in Fig.5(B) in der Größenordnung von 100 nm.

### Beispiel 4 Herstellung von trägerfixierten Polyelektrolythüllen

Ein sorgfältig gereinigter Glasträger wird 5 min in eine wäßrige Lösung von 0,5 mg/ml Polyethylenimin eingetaucht. Danach bläst man den Glasträger in einem Stickstoffstrom trocken. 100 µl einer 3%-igen Dispersion aus teilvernetzten Melaminformaldehydpartikeln mit einer Partikelgröße von 1 µm Durchmesser werden mit 400 µl einer 20 mono-mM Na-Poly(styrolsulfonat)-lösung NaCl versetzt. Nach 5 min unter leichtem Schütteln wird 1 ml reines Wasser zugesetzt. Nach Zentrifugation bei 2000 U/min wird der Überstand decantiert, das Sediment mit reinem Wasser aufgefüllt und die Zentrifugation wiederholt. Nach abermaligem Decantieren und einem weiteren Zentrifugationszyklus erhält man mit einer PSS-Schicht bedeckte MF-Partikel. Anschließend gibt man 400 µl einer 20 mono-mM-Polydiallyldimethylammoniumchloridlösung in 0,5 M NaCl zu den Partikeln und inkubiert für 20 min Diesen Vorgang wiederholt man ein zweites Mal. Anschließend werden die Partikel wiederum wie oben beschrieben mit PSS beschichtet und dreimal zentrifugiert. Das Sediment wird in 0,5 ml reinem Wasser redispergiert und auf den Glasträger aufgebracht. Nach 5 min taucht man den Glasträger für 5 min in eine 0,1 N Salzsäurelösung. Danach taucht man das Glasplättchen ohne Zwischentrocknung für jeweils 5 min dreimal in reines Wasser. Danach wird das Glasplättchen in einem leichten Stickstoffstrom getrocknet. Als Resultat erhält man dichtgepackte, auf einem Polyethylenimin-beschichteten Glasträger fixierte Polyelektrolythüllen, die aus 5 Schichten bestehen.

### Beispiel5 Herstellung von leeren Polyelektrolythüllen unter Verwendung biologischer Partikel als Templat

Rinder- oder Humanerythrozyten werden mit Glutadialdehyd in einer Konzentration von 2% fixiert. Nach 60 min Einwirkzeit bei 20°C wird die Lösung abzentrifugiert und die Erythrozyten werden viermal in bidestilliertem Wasser gewaschen. Anschließend werden die fixierten Erythrozyten mit ungepufferter 154 mM NaCl-Lösung aufgefüllt.

Zur Beschichtung werden 4 ml Lösung mit einer Konzentration von 0,5 g/dl PAH und 0,5 M NaCl bei einer Erythrozytenkonzentration von ca. 2,5% (v/v) angesetzt. Nach 10 min Einwirkzeit bei 20°C werden die Erythrozyten abzentrifugiert und zweimal in einer 154 mM NaCl Lösung gewaschen. Anschließend werden 4 ml Lösung mit einer Konzentration von 0,5 g/dl PSS und 0,5 m NaCl und einer Erythrozytenkonzentration von ca. 2,5% (v/v) angesetzt. Nach 10 min Einwirkzeit bei 20°C werden die Erythrozyten abzentrifugiert und zweimal in einer 154 mM NaCl-Lösung gewaschen. Das Aufbringen von PAH und PSS Schichten kann beliebig oft wiederholt werden.

Die Auflösung des Templats kann in einer 1,2%-igen NaOCl Lösung erfolgen. Ebenso geeignet sind handelsübliche Deproteinisierungsmittel (Medical Instruments) oder Abflußreiniger (z.B. Chlorix). Die Einwirkzeit beträgt ca. 20 min bei 20°C und läßt sich optisch über die verschwindende Trübung der Lösung kontrollieren. Die verbleibenden Polymerhüllen werden anschließend in NaCl-Lösung gewaschen.

Auf analoge Weise können auch E.coli oder Hefezellen beschichtet werden. Auch nichtfixierte Zellen können beschichtet werden.

### Beispiel 6 Einschluß von organischen Lösungsmitteln in Polyelektrolythüllen

Eine wässrige Suspension von Polyelektrolythüllen wird 5 min bei 3000 Upm zentrifugiert. Nach Entfernen des Überstands wird Methanol zugegeben. Die Hüllen werden resuspendiert und 10 min lang bei 4000 Upm zentrifugiert. Erneut wird der Überstand entfernt, Methanol zugegeben und die Probe unter denselben Bedingungen wie zuvor zentrifugiert. Diese Prozedur wird dreimal wiederholt. Nach der letzten Zentrifugation mit Methanol wird der Überstand durch Hexanol ersetzt. Die Hüllen werden resuspendiert und 10 min bei 5000 Upm zentrifugiert. Diese Prozedur wird wiederum dreimal wiederholt.

Zum Einschluß von Octanol, Octan oder Decan in die Hüllen wird eine ähnliche Prozedur verwendet, wobei als Ausgangsmaterial die in einer Hexanollösung vorliegenden Hüllen verwendet werden. Die Zentrifugationsgeschwindigkeit wird für Octanol und Octan auf 7000 Upm (10 min) und für Decan auf 7500 Upm (10 min) erhöht.

Schließlich wird das resultierende Sediment in Wasser resuspendiert. Die Hüllen verbleiben in der wässrigen Phase, während die noch vorhandenen Spuren des Lösungsmittels im Sediment zwischen den Hüllen eine zweite organische Phase bilden. Durch Verwendung von Fluoreszenzmarkern für die organische und die wässrige Phase kann mittels konfokaler Mikroskopie gezeigt werden, daß die Hüllen mit organischem Lösungsmittel gefüllt sind.

Die beschriebene Prozedur ermöglicht die Herstellung einer hochstabilen Emulsion von nichtpolaren Flüssigkeiten in Wasser. Als Folge der Monodispersität der ursprünglichen Hüllen ist die erzeugte Emulsion ebenso monodispers. Ein weiterer Vorteil ist, daß selbst die Form der einzelnen Tröpfchen - abhängig vom verwendeten Templat - reguliert werden kann. Dies ermöglicht die Herstellung von Emulsionen mit Oberfläche:Volumen-Verhältnissen, die von denjenigen einer Kugel verschieden sind.

### Beispiel 7 Präzipitation und Kristallisation in Polyelektrolythüllen

Die leeren Polyelektrolythüllen können auch zur kontrollierten Präzipitation oder Kristallisation organischer oder anorganischer Materialien verwendet werden. Hierzu werden Polyelektrolythüllen in einer 30 mM 6-Carbofluorescein (6-CF) Lösung bei pH 7 inkubiert. Anschließend wird der pH-Wert der Lösung rasch auf einen Wert von 3,5 verändert, bei dem 6-CF weitgehend unlöslich wird. Nach Inkubation über 1 bis 12 h wird eine Mischung von vollständig mit 6-CF gefüllten und leeren Hüllen erhalten. In weiteren Experimenten konnte Rhodamin B in Polyelektrolythüllen durch Erhöhung des pH-Werts präzipitiert werden.

Die Präzipitation von Wirkstoffen kann auch durch andere Maßnahmen, z.B. Lösungsmittelaustausch, Salzfällung, etc. ausgelöst werden. Diese Ergebnisse zeigen, daß die Polyelektrolythüllen als Template für Kristallisations- oder Präzipitationsprozesse dienen können, wodurch eine Kontrolle der Größe und Form von durch die Reaktion entstandenen kolloidalen Teilchen ermöglicht wird.

### Beispiel 8 Polymerisation in Polyelektrolythüllen

Eine 3% Lösung von Diallyldimethyldiammoniumchlorid (DADMAC) wird in einer 2%-igen Suspension von Polyelektrolythüllen mit dem Polymerisationsinitiator Natriumperoxodisulfat (30 mg/100ml) versetzt und 9,5 h bei 70°C polymerisiert. Durch Zentrifugation wird das in der Volumenphase synthetisierte Polymer PDADMAC abgetrennt. Durch Behandlung mit 100 mM 6-CF, das an die Aminogruppen von PDADMAC bindet, kann das Vorhandensein von an die negativ geladenen Kapselwände adsorbiertem Polymer und im Inneren der Kapsel befindlichem Polymer nachgewiesen werden.

Aus 9 Schichten bestehende Polyelektrolythüllen ([PSS/PAH]₄PSS) werden auf Humanerythrozyten abgeschieden und die Templatpartikel entfernt. Anschließend wird eine weitere Schicht PAH aufgetragen. Die Kapseln werden zur radikalischen Polymerisation von Acrylsäure zu Polyacrylsäure verwendet. Hierzu wird eine 3%ige Monomerlösung in einer 2%igen Kapselsuspension mit dem Initiator Natriumperoxodisulfat (30 mg/100 ml) versetzt und 9,5 h bei 70°C polymerisiert. Durch Zentrifugation wird die in der Volumenphase synthetisierte Polyacrylsäure entfernt. Nach Zugabe von 100 mM Rhodamin B, das selektiv an anionische Gruppen bindet, kann das Vorhandensein von Polyacrylsäure adsorbiert an die negativ geladenen Kapselwände, aber auch im Inneren der Kapseln nachgewiesen werden. Die Adsorption von Acrylsäure an Kapselwände kann durch Verwendung von Kapseln mit einer äußeren negativen Ladung verhindert werden.

### Beispiel9 Kolloidale Stabilisierung von mit organischen Lösungsmitteln gefüllten Polyelektrolythüllen

Polyelektrolythüllen in wässriger Lösung werden mit DPPA (unter Zusatz von 5% markiertem DPPC) beschickt. Die wässrige Dispersion wird mit Pentanol, Octanol oder Decan gemischt. Die Proben werden für 2 min bei 17.000 Upm zentrifugiert. Die Mischung trennt sich in zwei Phasen auf, wobei die Hüllen an der Zwischenschicht zwischen der wässrigen und der organischen Phase lokalisiert sind. Die wässrigen Phase wird extrahiert und die Probe dreimal mit dem organischen Lösungsmittel gewaschen.

Durch konfokale Mikroskopie kann nachgewiesen werden, daß die Lipide selbst nach Kontakt mit dem organischen Lösungsmittel an den Hüllen verbleiben und daß im Inneren der Hülle eine wäßrige Phase verkapselt ist. Hierzu werden die Kapseln vor dem Aufbringen der Lipidschicht 1 h in einer 0,1 mM 6-CF-Lösung inkubiert und die Probe nach Aufbringen der Lipide viermal mit Wasser zur Entfernung von überschüssigen Lipiden und 6-CF gewaschen. Ein 12 h nach der Präparation aufgenommenes konfokales Bild zeigt, daß die wässrige 6-CF-Lösung immer noch innerhalb der Hüllen verkapselt ist. Das organische Lösungsmittel zeigt keine Fluoreszenz.

Diese Ergebnisse zeigen, daß die Polyelektrolythüllen zur Verkapselung organischer Lösungsmittel in Wasser und auch umgekehrt zur Verkapselung einer wässrigen Phase im organischen Medium verwendet werden können. Somit können stabile Öl-in-Wasser- und Wasser-in-Öl-Emulsionen ohne Verwendung oberflächenaktiver Mittel erzeugt werden.

### Beispiel 10 Permeabilität von Polyelektrolythüllen

Polyelektrolythüllen wurden durch Beschichtung von 3 µm Melaminformaldehydpartikeln unter Verwendung von Natriumpoly(styrolsulfonat) mit einem Molekulargewicht von 70.000 und Poly(allylaminhydrochlorid) mit einem Molekulargewicht von 50.000 erzeugt. Für die konfokale Fluoreszenzmikroskopie wird ein mit Fluoresceinisothiocyanat markiertes PAH (FITC-PAH) eingesetzt (Sukhorukov, Colloids Surfaces A 137 (1998), 253).

Suspensionen von Melaminformaldehydpartikeln mit 3 µm Durchmesser werden mit 13 Schichten PAH und PSS beschichtet. Dann wird durch 5 min Inkubation in 0,1 NaCl der Kern aufgelöst.

Mittels konfokaler Mikroskopie wird die Permeabilität der Polyelektrolytmikrokapseln untersucht. Hierzu wird zunächst eine Lösung von PAH-FITC (Molekulargewicht 50.000) mit den Hüllen auf eine Endkonzentration von 0,5 mg/ml vermischt. Die Permeabilität der Hüllwände für hochmolekulares PAH-FITC ist so gering, daß nach 20 min Inkubation keine Fluoreszenz im Inneren der Kapseln nachweisbar war. Im Gegensatz dazu kann 6-Carbofluoreszein (6-CF) die Wände der Hüllen ohne weiteres durchdringen.

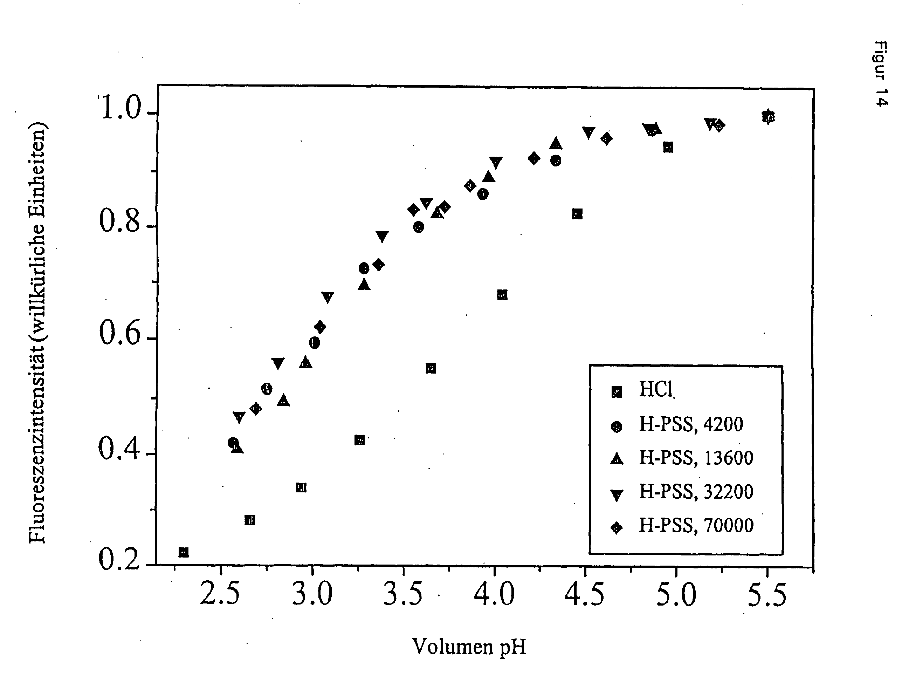

Figur 14 zeigt die Fluoreszenzintensität einer Suspension von 6-CF als Fluoreszenzmarker enthaltenden Polyelektrolytkapseln gegenüber den durch Polystyrolsulfonsäure und HCl titrierten pH-Wert im umgebenden Medium. Die Ergebnisse zeigen, daß Polystyrolsulfonsäure in den jeweils verwendeten Molekulargewichten (70.000-4.200) die Polyelektrolythüllen nicht durchdringen konnte.

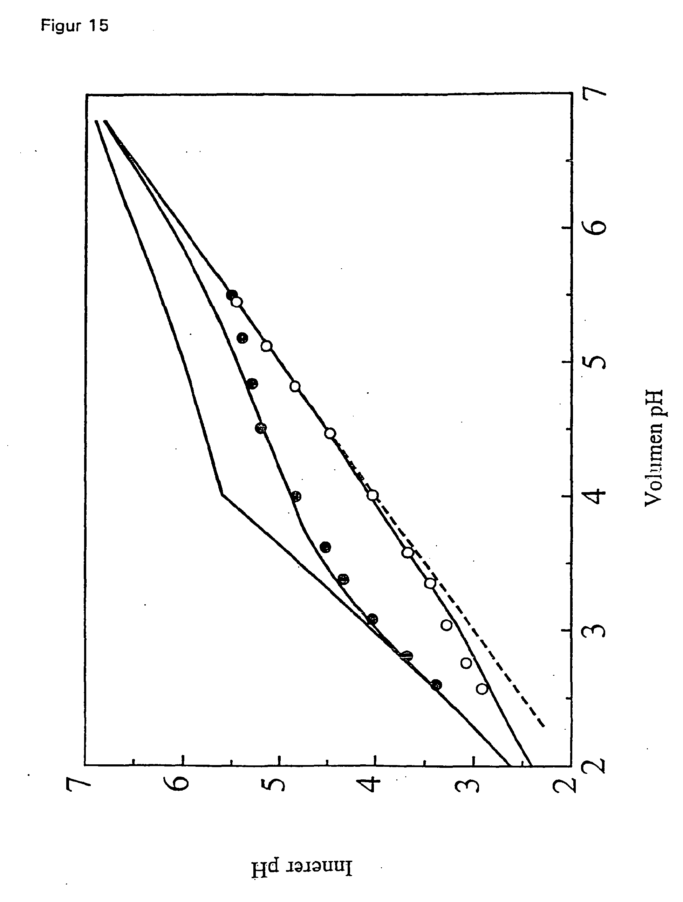

Figur 15 zeigt den pH-Wert im Inneren der Kapseln als Funktion des mit H-PSS (Molekulargewicht 4200) titrierten Volumen pH-Werts in Abwesenheit von Salz und in Gegenwart von 1 mM NaCl. Es ist zu erkennen, daß mindestens innerhalb einer Stunde keine signifikante Diffusion von PSS Polyanionen mit dem jeweils getesteten Molekulargewichten ins Innere der Kapsel stattfindet. Dies führt zum Entstehen eines pH-Gradienten zwischen dem Inneren der Kapsel und dem Volumen. Der pH-Wert im Inneren ist um etwa eine pH-Einheit basischer als der pH-Wert des Volumens. Dies gilt insbesondere für einen Volumen pH-Wert geringer als 5,5.

## Patentansprüche

1. Verfahren zur Herstellung von Kapseln mit einer Polyelektrolythülle, die mehrere Polyelektrolytschichten umfasst, und einen Durchmesser bis zu 10 µm aufweist, umfassend die Schritte:
a) Bereitstellen einer wässrigen Dispersion von Templatpartikeln geeigneter Größe,
b) Herstellen einer Hülle um die Partikel durch Aufbringen von Polyelektrolyten auf die Templatpartikel und
c) zumindest teilweise Desintegration der Templatpartikel.

2. Verfahren nach Anspruch 1,
**dadurch gekennzeichnet,**
**dass** zwei bis vierzig Polyelektrolytschichten aufgebracht werden.

3. Verfahren nach einem der Ansprüche 1 oder 2,
**dadurch gekennzeichnet,**
**dass** nacheinander Schichten von jeweils entgegengesetzt geladenen Polyelektrolyten auf die Templatpartikel aufgebracht werden.

4. Verfahren nach einem der vorhergehenden Ansprüche,
**dadurch gekennzeichnet,**
**dass** die Desintegration der Templatpartikel durch eine Veränderung des pH-Werts oder/und Sulfonierung der Templatpartikel bewirkt wird.

5. Verfahren nach einem der Ansprüche 1 bis 3,
**dadurch gekennzeichnet,**
**dass** die Desintegration des Templats durch Zugabe eines Lysereagenz erfolgt.

6. Verfahren nach Anspruch 5,
**dadurch gekennzeichnet,**
**dass** das Lysereagenz ein Deproteinisierungsmittel ausgewählt aus Peroxo- und Hypochloritverbindungen enthält.

7. Verfahren nach Anspruch 6,
**dadurch gekennzeichnet, dass** man Natrium- oder Kaliumhypochlorit als Deproteinisierungsmittel verwendet.

8. Verfahren nach einem der vorhergehenden Ansprüche, weiterhin umfassend den Schritt:
d) Abtrennen von Templatpartikel-Fragmenten.

9. Kapsel, erhältlich durch ein Verfahren nach einem der vorherigen Ansprüche
**dadurch gekennzeichnet,**
**dass** sie eine von den Templatpartikeln vorgegebene äußere Form aufweist.

10. Kapsel nach Anspruch 9,
**dadurch gekennzeichnet,**
**dass** sie eine anisotrope Form aufweist.

11. Kapsel nach Anspruch 9 oder 10,
**dadurch gekennzeichnet,**
**dass** die Hülle Poren enthält.

12. Kapsel nach Anspruch 11,
**dadurch gekennzeichnet,**
**dass** die Poren den Durchtritt von Substanzen mit einem Molekulargewicht ≤ 4 kD erlauben.

13. Kapsel nach einem der Ansprüche 9 bis 12,
**dadurch gekennzeichnet,**
**dass** die Hülle weiterhin Nanopartikel, Tenside, Proteine oder/und Enzyme enthält.

14. Kapsel nach einem der Ansprüche 9 bis 13,
**dadurch gekennzeichnet,**
**dass** innerhalb der Hülle eine Flüssigphase vorliegt.

15. Kapsel nach einem der Ansprüche 9 bis 14,
**dadurch gekennzeichnet,**
**dass** sie einen Durchmesser < 5 µm aufweist.

16. Kapsel nach einem der Ansprüche 9 bis 14,
**dadurch gekennzeichnet,**
**dass** die Stärke der Hüllwand 2 bis 100 nm beträgt.

17. Zusammensetzung,
**dadurch gekennzeichnet,**
**dass** sie mehrere Kapseln nach einem der Ansprüche 9 bis 16 mit monodisperser Größenverteilung enthält.

18. Zusammensetzung,
**dadurch gekennzeichnet,**
**dass** sie Kapseln nach einem der Ansprüche 9 bis 16 in getrockneter, eingefrorener oder gefriergetrockneter Form enthält.

19. Verwendung von Kapseln nach einem der Ansprüche 9 bis 16
als Reaktionsraum, zur Herstellung von Kristallen, zum Aufbau von Mikro- bzw. Nanokompositen, als Emulsionsträger, zum Einbringen von organischen Flüssigkeiten oder zum Verkapseln von Gasen.

20. Verwendung von Kapseln nach einem der Ansprüche 9 bis 16 in der Sensorik, Oberflächenanalytik, Informationstechnologie, in der Pharmazie und Medizin, Lebensmitteltechnologie, Biotechnologie, und Kosmetik.

## Claims

1. A method for producing capsules, with a polyelectrolyte shell comprising a plurality of polyelectrolyte layers and a diameter of up to 10 µm, comprising the steps of:
a) providing an aqueous dispersion of template particles of suited size,
b) producing a shell surrounding the particle by applying polyelectrolytes on the template particle, and
c) at least partially disintegrating the template particle.

2. Method according to claim 1, **characterized in that** 2 to 40 polyelectrolyte layers are applied.

3. Method according to anyone of claims 1 or 2, **characterized in that** alternating layers of oppositely charged polyelectrolytes are applied onto the template particle.

4. Method according to anyone of the preceding claims, **characterized in that** the disintegration of the template particles is caused by a change of the pH-value or/and sulfonation of the template particle.

5. Method according to anyone of claims 1 to 3, **characterized in that** disintegration of the template is carried out through addition of a lysis reagent.

6. Method according to claim 5, **characterized in that** the lysis reagent is a deproteinizing agent selected from peroxo- and hypochlorite compounds.

7. Method according to claim 6, **characterized in that** sodium- or potassium hypochlorite are used as deproteinizing agent.

8. Method according to anyone of the preceding claims, further comprising the step:
d) separation of template particle fragments.

9. A capsule obtainable by a method according to anyone of the preceding claims, **characterized in that** they have an outer shape which is given by the template particle.

10. A capsule according to claim 9, **characterized in that** it has an anisotropic shape.

11. A capsule according to claim 9 or 10, **characterized in that** the shell comprises pores.

12. A capsule according to claim 11, **characterized in that** the pores permit substances with a molecular weight of ≤4 kD to pass through.

13. A capsule according to anyone of claims 9 to 12, **characterized** that the shell further comprises nano particles, surfactants, proteins or/and enzymes.

14. A capsule according to anyone of claims 9 to 13, **characterized in that** a liquid phase is present inside the shell.

15. A capsule according to anyone of claims 9 to 14, **characterized in that** it has a diameter of < 5 µm.

16. A capsule according to anyone of claims 9 to 14, **characterized in that** the thickness of the shell is between 2 to 100 nm.

17. A composition, **characterized in that** it comprises several capsules according to anyone of claims 9 to 16 with a monodisperse size distribution.

18. Composition, **characterized in that** it comprises capsules according to anyone of claims 9 to 16 in dried, frozen or freeze-dried form.

19. Use of a capsule according of anyone of claims 9 to 16 as a reaction chamber for the production of crystals, for the assembly of micro- respectively nano compositions, as an emulsion carrier, for the application of organic liquids or for encapsulating gases.

20. Use of capsules according to anyone of claims 9 to 16 in sensor technology, surface analytics, information technology, in pharmacy and medicine, food technology, biotechnology and cosmetics.

## Revendications

1. Procédé de fabrication de capsules ayant une enveloppe de polyélectrolyte, qui comporte plusieurs couches d'électrolyte et qui présente un diamètre allant jusqu'à 10 µm, comprenant les étapes suivantes :
a) apprêt d'une dispersion aqueuse de particules modèles d'une taille appropriée,
b) fabrication d'une enveloppe autour de la particule par application de polyélectrolytes sur la particule modèle et
c) désintégration au moins partielle de la particule modèle.

2. Procédé selon la revendication 1, **caractérisé en ce que** l'on applique deux à quarante couches de polyélectrolyte.

3. Procédé selon l'une quelconque des revendications 1 ou 2, **caractérisé en ce que** l'on applique sur la particule modèle les unes après les autres des couches de polyélectrolytes à charge opposée.

4. Procédé selon l'une quelconque des revendications précédentes, **caractérisé en ce que** la désintégration de la particule modèle est effectuée par une modification de la valeur de pH et/ou par la sulfonation de la particule modèle.

5. Procédé selon l'une quelconque des revendications 1 à 3, **caractérisé en ce que** la désintégration du modèle se fait par addition d'un réactif de lyse.

6. Procédé selon la revendication 5, **caractérisé en ce que** le réactif de lyse contient un agent de déprotéinisation sélectionné parmi des composés de peroxydes et d'hypochlorites.

7. Procédé selon la revendication 6, **caractérisé en ce que** l'on utilise en tant qu'agent de déprotéinisation l'hypochlorite de sodium ou de potassium.

8. Procédé selon l'une quelconque des revendications précédentes, comprenant en outre l'étape :
(d) séparation des fragments de particule modèle.

9. Capsule, que l'on peut obtenir par un procédé selon l'une quelconque des revendications précédentes, **caractérisée en ce qu'**elle présente une forme externe prédéterminée par les particules modèle.

10. Capsule selon la revendication 9, **caractérisée en ce qu'**elle présente une forme anisotrope.

11. Capsule selon la revendication 9 ou 10, **caractérisée en ce que** l'enveloppe contient des pores.

12. Capsule selon la revendication 11, **caractérisée en ce que** les pores permettent le passage de substances ayant un poids moléculaire ≤4 kD.

13. Capsule selon l'une quelconque des revendications 9 à 12, **caractérisée en ce que** l'enveloppe contient en outre des nanoparticules, des agents tensioactifs, des protéines et/ou des enzymes.

14. Capsule selon l'une quelconque des revendications 9 à 13, **caractérisée en ce qu'**est présente au sein de l'enveloppe une phase liquide.

15. Capsule selon l'une quelconque des revendications 9 à 14, **caractérisée en ce qu'**elle présente un diamètre < 5 µm.

16. Capsule selon l'une quelconque des revendications 9 à 14, **caractérisé en ce que** l'épaisseur de la paroi de l'enveloppe va de 2 à 100 nm.

17. Composition, **caractérisée en ce qu'**elle contient plusieurs capsules selon l'une quelconque des revendications 9 à 16 ayant une répartition granulométrique monodispersée.

18. Composition, **caractérisée en ce qu'**elle contient des capsules selon l'une quelconque des revendications 9 à 16 sous une forme séchée, congelée ou lyophilisée.

19. Utilisation de capsules selon l'une quelconque des revendications 9 à 16 en tant que chambre de réaction en vue de la fabrication de cristaux, de la formation de substances micro- ou nano-composites, en tant que vecteur d'émulsion, en vue de l'introduction de liquides organiques ou de l'encapsulation de gaz.

20. Utilisation de capsules selon l'une quelconque des revendications précédentes 9 à 16, dans la technique des systèmes capteurs, dans l'analytique des surfaces, en technologie informatique, en pharmacie et en médecine, en technologie des produits alimentaires, en biotechnologie et dans l'industrie des produits cosmétiques.
